(19) **Europäisches Patentamt / European Patent Office / Office européen des brevets**

(11) **EP 3 280 497 B1**

(12) **FASCICULE DE BREVET EUROPEEN**

(45) Date de publication et mention de la délivrance du brevet:
**26.02.2020 Bulletin 2020/09**

(51) Int Cl.:
**A61K 8/9783** (2017.01) **A61Q 17/00** (2006.01)
**A61Q 19/08** (2006.01)

(21) Numéro de dépôt: **16713951.8**

(22) Date de dépôt: **05.04.2016**

(86) Numéro de dépôt international:
**PCT/EP2016/057457**

(87) Numéro de publication internationale:
**WO 2016/162343 (13.10.2016 Gazette 2016/41)**

(54) **EXTRAIT HYDRO-ALCOOLIQUE DE SCHINUS MOLLE, COMPOSITIONS COSMETIQUES LE COMPRENANT ET LEURS UTILISATIONS COSMETIQUES**

HYDROALKOHOLISCHES EXTRAKT AUS SCHINUS MOLLE, KOSMETISCHE ZUSAMMENSETZUNGEN DAMIT UND KOSMETISCHE VERWENDUNGEN DAVON

HYDROALCOHOLIC EXTRACT OFSCHINUS MOLLE, COSMETIC COMPOSITIONS COMPRISING THE SAME AND COSMETIC USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.04.2015 FR 1553082**

(43) Date de publication de la demande:
**14.02.2018 Bulletin 2018/07**

(73) Titulaires:
• **ISP Investments LLC**
**Wilmington, DE 19805 (US)**
• **Jafer Enterprises R&D, S.L.**
**(Sociedad Unipersonal)**
**08403 Granollers (Barcelona) (ES)**

(72) Inventeurs:
• **IMBERT, Isabelle**
**06400 Cannes (FR)**
• **DOMLOGE, Nouha**
**06650 Opio (FR)**
• **BOTTO, Jean Marie**
**06560 Valbonne (FR)**
• **LAVILLE, Rémi**
**06340 Drap (FR)**
• **GARNIER, Sébastien**
**06650 Le Rouret (FR)**

(74) Mandataire: **Axe PI**
**7, place Ile de Beauté**
**06300 Nice (FR)**

(56) Documents cités:
AR-A1- 079 996        JP-A- 2000 247 862
US-A1- 2013 302 419

• **MARZOUK ET AL: "Antioxidant flavonol glycosides from Schinus Molle", PHYTOTHERAPY RESEARCH, vol. 20, 2006, pages 200-205, XP002749056,**
• **"International Cosmetic Ingredient Dictionary and Handbook", 2014, XP002749057, vol. 2, page 3188, alinéa [id20187] alinéa [id18740] alinéa [id28467] alinéa [id22338]**
• **Anonymous: "Pink pepper extract 136825 (ingredients)", Personal Care Products Council , 26 octobre 2015 (2015-10-26), XP002749058, Extrait de l'Internet: URL:http://webdictionary.personalcarecounc il.org/jsp/MixtureDetailPage.jsp?ID=384167 &type=M [extrait le 2015-10-26]**
• **Anonymous: "schinus molle extract (ingredients)", Personal Care Products Council , 26 octobre 2015 (2015-10-26), XP002749059, Extrait de l'Internet: URL:http://webdictionary.personalcarecounc il.org/jsp/MixtureDetail.jsp?ID=399294&typ e=M [extrait le 2015-10-26]**

- Anonymous: "Elixiance biofunctional NAtural and sustainable Perusian Schinus molle extract with anti-pollution, skin-purifying age-perfectin benefits", Ashland , 2015, XP002749060, Extrait de l'Internet: URL:https://www.ulprospector.com/documents /1392972.pdf?bs=4989&b=585194&st=20 [extrait le 2015-10-26]
- Anonymous: "Vincience Biofunctionals Skin care and hair care applications.", Ashland , 2015, pages FP-20, XP002749061, Extrait de l'Internet: URL:https://www.ulprospector.com/documents /1183059.pdf?bs=4989&b=585194&st=20 [extrait le 2015-10-26]
- Anonymous: "Innovation zone 2015", in-cosmetics , 2015, pages FP-49, XP002749062, Extrait de l'Internet: URL:http://www.in-cosmetics.com/RXUK/RXUK _ InCosmetics/2015-Website/Documents/8793_IN COS_EUROPE_INNOVATION_ZONE_GUIDE_20 15_v16_ LO.pdf?v=635666863387152785 [extrait le 2015-10-26]

**Description**

L'invention concerne un extrait hydro-alcoolique de *Schinus molle,* son procédé de préparation, des compositions cosmétiques le comprenant et leurs utilisations cosmétiques.

Le *Schinus molle,* également communément appelé « faux-poivrier » ou « poivrier sauvage » ou encore « arbre à poivre de Californie » ou « poivrier du Pérou », est un arbre de la famille des Anacardiacées originaire de la zone aride du nord de l'Amérique du Sud et des déserts andins du Pérou, et se retrouve jusqu'en Argentine et au Chili. Il a également été introduit dans la plupart des régions tropicales et subtropicales du monde.

*Schinus molle* est un arbre à croissance rapide, à feuilles persistantes, de 15 mètres de hauteur et de 5 à 10 mètres de largeur. Les branches supérieures ont particulièrement tendance à retomber. Les feuilles sont disposées de façon alterne sur les rameaux. Elles mesurent généralement 8-25 cm de long et 4-9 cm de large, sont finement découpées, de forme imparipennée, composées de nombreuses folioles au contour entier et à nervation pennée, et dégagent une forte odeur de poivre lorsqu'elles sont froissées. Sa floraison est printanière avec des fleurs petites, en grappes pendantes, de couleur blanche, situées aux extrémités des branches et qui donnent naissance à des baies roses de 5-7 mm de diamètre à odeur poivrée. Son tronc tortueux et noueux présente une écorce rugueuse de couleur grisâtre qui dégage également une odeur poivrée.

En médecine traditionnelle, *Schinus molle* a notamment été utilisé pour ses propriétés antibactériennes et antiseptiques dans le traitement des plaies et des infections (Ferreroa et al., "Acute and subacute toxicity evaluation of ethanolic extract from fruits of Schinus molle in rats", 2007). Il apparait également avoir été utilisé comme antidépresseur (Machado et al., "Antidepressant-like effect of rutin isolated from the ethanolic extract from Schinus molle L. in mice: Evidence for the involvement of the serotonergic and noradrenergic systems", 2008), diurétique, contre les maux de dents, les rhumatismes et les troubles menstruels. Les fruits, les feuilles et la sève de *Schinus molle* ont ainsi été utilisés pour leurs propriétés anti-hypertensives, diurétiques, antispasmodiques et analgésiques (Ambasata et al., « The useful plants of India », 1986 ; Bello et al., « In vitro pharmacological evaluation of the dichloromethanol extract from Schinus molle L. », 1998).

Les propriétés insecticides de *Schinus molle* permettent de le considérer comme une alternative aux produits chimiques synthétiques dans la lutte antiparasitaire (Ferreroa et al., "Acute and subacute toxicity evaluation of ethanolic extract from fruits of Schinus molle in rats", 2007). A cet effet, l'activité insecticide d'extraits aqueux et éthanoliques de feuilles de *Schinus molle* a été testée sur la Galéruque de l'orme (Huerta et al., « Toxicity and repellence of aqueous and ethanolic extracts from Schinus molle on elm leaf beetle Xanthogaleruca luteola », 2010). On notera que l'extrait aqueux et l'extrait éthanolique ont des effets strictement différents avec un effet répulsif pour l'extrait aqueux alors que l'extrait éthanolique présente un effet insecticide.

D'autres publications concernent les propriétés antioxydantes, anti microbiennes et toxicologiques de l'huile essentielle de *Schinus molle* (par exemple Martins Mdo et al., "antimicrobial and toxicological properties of Schinus molle L. essential oils", 2013).

Des extraits éthanoliques et hexaniques de feuilles de *Schinus molle* ont plus particulièrement été testés pour leur toxicité sur des rats afin d'évaluer leur toxicité cutanée (Bras et al., « Evaluation of the acute dermal exposure of the ethanolic and hexanic extracts from leaves of Schinus molle var. areira in rats », 2011). Une légère et réversible irritation cutanée a ainsi été observée.

Le document brevet US2013302419 divulgue qu'un extrait méthanolique ou éthanolique pur de plante de la famille des Anacardiaceae, parmi lesquelles le Schinus Molle, permet de préparer une composition pharmaceutique utilisée dans le traitement des pathologies du tractus digestif. Les parties de la plante pouvant être utilisées sont les feuilles, les fleurs, les fruits et préférentiellement l'écorce. Les extraits obtenus contiennent principalement de l'acide gallique.

Le document de brevet AR 079996 décrit la préparation de colorants végétaux à partir de diverses plantes dont les fruits de Schinus molle. L'extraction peut se faire à l'aide de solvant miscible dans l'eau, comme le méthanol, l'éthanol ou l'isopropanol.

Le dictionnaire des ingrédients cosmétiques (International Cosmetic Ingredient Dictionary and Handbook 2014)cite un extrait total, un extrait de feuille, un extrait de fruit, un extrait de graine de Schinus molle, cependant cette référence ne précise pas les conditions d'extraction ni les effets cosmétiques précis des ingrédients cités.

Les principaux composés qui ont été isolés de *Schinus molle* sont des cétoacides triterpéniques (Pozzo-Balbi et al., « The triterpenoid acids of Schinus molle », 1978). Un certain nombre de mono-, di-, sesqui- et triterpènes, de flavonoïdes, de gallotannins et d'acides gras ont été rapportés chez *Schinus molle* (Hänsel et al., « Hagers Handbuch der pharmazeutischen praxis », 1994; Terhune et al, « β-spathulene : a new sesquiterpene in Schinus molle oil », 1974; Dominguez et al., « Lignoceric acid and other compounds of Schinus molle », 1971). Une autre étude a conduit à l'isolement de 3 agents anti-inflammatoires, 2 acides triterpéniques et une biflavanone (Yueqin et al., « Isolation of two triterpenoids and a biflavanone with an anti-inflammatory activity from Schinus molle fruits », 2003).

Un criblage 2D-PC d'un extrait obtenu par extraction dans un mélange eau et 80% méthanol des feuilles de *Schinus molle* a mis en évidence un mélange complexe de flavonols glycosylés et plus particulièrement des dérivés glycosylés de la quercétine (Hiermann et al., « Die Untersuchung potentieller Wirkstoffe in epilobium Arten. », 1983). La publication Marzouk et al. (« Antioxidant flavonol glycosides from Schinus molle », 2006) a plus particulièrement mis en évidence à partir d'un extrait aqueux 80% méthanol des feuilles de *Schinus molle* par séparation chromatographique, 2 nouveaux glycosides quercétine acylés : l'isoquercitrin 6"-O-p-hydroxybenzoate et la 2"-O-alpha-L-rhamnopyranosyl-hyperin 6"-O-gallate, ainsi que 12 métabolites polyphénols connus dans cette espèce, à savoir l'acide gallique, le méthyl gallate, l'acide chlorogénique, la 2"-alpha-L-rhamnopyranosyl-hypérine, la quercétine 3-O-beta-D-neohesperidoside, la miquelianine, la quercétine 3-O-beta-D-galacturonopyranoside, l'isoquercitrine, l'hyperine, l'isoquercitrine 6"-gallate, l'hyperine 6"-O-gallate et la (+)-catechine.

De nombreuses publications concernent ainsi différents extraits de *Schinus molle* mais aucune ne semble divulguer ni même suggérer une utilisation cosmétique, et plus particulièrement pour améliorer la fonction barrière de la peau et protéger contre les polluants atmosphériques, et ainsi par exemple lutter contre l'apparition des signes du vieillissement cutané.

On connait par ailleurs le document brevet JP04176912. Il décrit une composition comprenant un ou plusieurs extraits de plantes choisies parmi Aguaje (*Mauritia flexuosa L. f., Achira*), (*Canna edulis Ker Gawl.*), Alg arrobo (*Prosopis pallida DC.*), Huito (*Genipa americana L.*), Oca (*Oxalis tuberosa Mo I.*), Olluco (*Ullucus tuberousus Caldas*), Cupuazu (*Theobroma grandiflorum K. Schu m.*), Tarwi (*Lupinus mutabilis Sweet*), Maca (*Lepidium meyenii Walpers*), Mashua (*Trop aeolum tuberosum Ruizet Pav.*), Molle (*Schinus molle L.*) et Yacon (*polymnia sonchi folia Poepp.* et *Endl.*). De telles compositions présentent un effet sur la rétention d'eau pour prévenir contre par exemple la sécheresse cutanée, de limiter les démangeaisons et conférer de la brillance à la peau et aux cheveux.

Toutefois, les solvants utilisés pour une macération à température ambiante pendant 3-7 jours sont ceux habituellement utilisés par l'homme du métier et sont plus particulièrement choisis parmi l'eau, l'éthanol, le 1,3-butylène glycol, le propylène glycol, le glycérol, et le polyéthylène glycol.

On connait encore le document brevet JP2000247862 qui décrit la préparation d'un extrait de plante entière de Pirul (Schinus molle) par macération pendant 5 jours dans un mélange eau-éthanol 50/50. Les extraits obtenus présentent des propriétés antioxydantes et peuvent être utilisés comme produits pharmaceutiques ou cosmétiques par exemple pour blanchir la peau.

Considérant ce qui précède, un problème que se propose de résoudre l'invention est de développer des produits à base d'un extrait de *Schinus molle* qui soient utilisables en cosmétique, faciles à mettre en œuvre, en optimisant les constantes physico-chimiques présentant des propriétés bénéfiques pour la peau tout en limitant les effets indésirables (toxicité) observés dans l'art antérieur.

En particulier, les Demandeurs ont développé un procédé d'extraction de *Schinus molle* qui a été optimisé pour sélectionner et garantir dans l'extrait obtenu une forte teneur en molécules cibles d'intérêt, et spécifiquement 2 polyphénols d'intérêt dont un n'a encore jamais été décrit dans *Schinus molle*. Ce développement a été réalisé selon des critères d'ecoextraction (solvants biosourcés, optimisation de température de chauffage et temps d'agitation, rapport quantité matière végétale / quantité et type de solvant) pour obtenir le meilleur compromis teneur polyphénolique / coût énergétique (le solvant étant avantageusement conservé dans l'extrait liquide), et afin d'augmenter la concentration en composés d'intérêt par rapport aux autres polyphénols extraits au regard des extraits connus de l'art antérieur.

Parmi les types d'extraction citées dans l'art antérieur, à savoir des extractions aqueuse, méthanolique, éthanolique, hexanique ou encore par entrainement à la vapeur (huile essentielle) ou extraction au $CO_2$ supercritique de *Schinus molle,* aucun ne permet l'extraction sélective des composés phénoliques d'intérêt selon l'invention.

En effet, l'extraction au $CO_2$ supercritique permet l'extraction de la phase huileuse et non pas des polyphénols d'intérêt. De même, une extraction aqueuse extrait majoritairement les composés polaires tels que les sucres, protéines et métabolites polaires de faible poids moléculaire, alors que les polyphénols d'intérêt sont présents seulement sous forme de trace, en raison de leur polarité. Enfin, les solvants alcooliques tels que le méthanol et l'éthanol extraient de façon non sélective des molécules polaires et apolaires présentes dans *Schinus molle* telles que des flavonols glycosides (isoquercitrine-6"-O-p-hydroxybenzoate, 2"-O-$\alpha$-L-rhamnopyranosyl-hyperine-6"-O-gallate, acide gallique, méthyle gallate, acide chlorogenique, 2"-$\alpha$-L-rhamnopyranosyl-hyperine, quercétine-3-O-$\beta$-D-neohesperidoside, miquelianine, quercétine-3-0-$\beta$-D-galacturonopyranoside, isoquercitrine, hyperine, isoquercitrine 6"-gallate, hyperine 6"-O-gallate, (+)-catechine), huile essentielle, triterpene stéroïdien, preisocalamenediol, acides gras (linoleique, linolenique, behenique, acides lignoceriques), insaponifiable ($\alpha$-amyrin, $\beta$-sitosterol), sucres (laccase : arabinose, xylose, mannose, galactose, glucose, glucosamine).

L'invention a donc pour premier objet un extrait hydro-alcoolique des parties aériennes de *Schinus molle* caractérisé en ce qu'il comprend majoritairement de la quercitrine et de la miquelianine parmi les polyphénols extraits, en une concentration respectivement d'au moins 0,04% de quercitrine en poids du poids total de l'extrait et au moins 0,02% de miquelianine en poids du poids total de l'extrait, les autres polyphénols extraits pris indépendamment les uns des autres étant présents sous forme de traces, c'est-à-dire à une concentration pour chaque polyphénol extrait pris indépendamment inférieure à 0,01% (100 ppm) en poids du poids total de l'extrait.

De plus, l'invention a pour deuxième objet un procédé d'obtention d'un extrait de *Schinus molle* selon l'invention, comprenant les étapes suivantes selon lesquelles :

a) on recueille les feuilles non débarrassées des petites tiges portant les feuilles de *Schinus molle* ;

b) on disperse les feuilles non débarrassées des petites tiges portant les feuilles de *Schinus molle* recueillies, séchées et broyées

dans une proportion de 5% à 10% en poids de matière solide par rapport au poids total mis en œuvre, dans un solvant hydro-alcoolique, l'alcool étant choisi parmi l'éthanol ou le propanediol, dans une proportion comprise entre 60% et 80% en poids d'alcool par rapport au poids total du solvant ;

c) on réalise une extraction solide-liquide, sous agitation, à une température comprise entre 20°C et 70°C pendant une durée comprise entre 1 heure et 2 heures ;

d) on sépare les phases liquide et solide de manière à éliminer la phase solide et récupérer un extrait hydro-alcoolique de *Schinus molle* liquide comprenant majoritairement de la quercitrine et de la miquelianine parmi les polyphénols extraits, en une concentration respectivement d'au moins 0,04% de quercitrine en poids du poids total de l'extrait et au moins 0,02% de miquelianine en poids du poids total de l'extrait, les autres polyphénols extraits pris indépendamment les uns des autres étant présents sous forme de traces, c'est-à-dire à une concentration pour chaque polyphénol extrait pris indépendamment inférieure à 0,01% (100 ppm) en poids du poids total de l'extrait ; et

e) éventuellement, lorsque l'alcool est l'éthanol, on sèche l'extrait de *Schinus molle* liquide obtenu de manière à obtenir un extrait de *Schinus molle* solide.

L'invention a pour troisième objet une composition cosmétique, caractérisée en ce qu'elle comprend, à titre d'agent actif protecteur, une quantité efficace d'un extrait de *Schinus molle* selon l'invention, et un excipient physiologiquement acceptable.

Enfin, l'invention a pour quatrième objet l'utilisation cosmétique d'une composition selon l'invention, pour améliorer la fonction barrière de la peau et protéger la peau contre les polluants atmosphériques.

L'invention et les avantages qui en découlent seront mieux compris à la lecture de la description et des modes de réalisation non limitatifs qui suivent, rédigée au regard des figures annexées dans lesquelles :

- la Figure 1 représente l'évaluation de l'effet protecteur d'un extrait de *Schinus molle* selon l'invention sur des kératinocytes soumis à un stress environnemental par des particules polluantes PM10-like ;
- la Figure 2 représente l'évaluation de l'effet protecteur d'un extrait de *Schinus molle* selon l'invention sur des kératinocytes soumis à un stress environnemental par des particules polluantes PM2.5-like ;
- la Figure 3 représente l'effet d'un extrait de *Schinus molle* selon l'invention sur la régulation de la sécrétion de sébum par mesure de la surface des spots ;
- la Figure 4 représente l'évolution de la sécrétion de sébum par mesure du nombre de spots après application d'un extrait de *Schinus molle* selon l'invention ;
- la Figure 5 représente l'effet d'un extrait de *Schinus molle* selon l'invention sur l'évolution du nombre de rides ;
- la Figure 6 représente l'effet d'un extrait de *Schinus molle* selon l'invention sur l'évolution de la profondeur moyenne des rides ;
- la Figure 7 représente l'effet d'un extrait de *Schinus molle* selon l'invention sur l'évolution de l'hydratation de la peau ; et
- la Figure 8 représente l'effet hydratant d'un extrait de *Schinus molle* selon l'invention.

Dans cette description, à moins qu'il ne soit spécifié autrement, il est entendu que, lorsqu'un intervalle est donné, il inclut les bornes supérieure et inférieure dudit intervalle.

L'invention concerne un extrait hydro-alcoolique des parties aériennes de *Schinus molle* comprenant majoritairement de la quercitrine et de la miquelianine parmi les polyphénols extraits, en une concentration respectivement d'au moins 0,04% de quercitrine en poids du poids total de l'extrait et au moins 0,02% de miquelianine en poids du poids total de l'extrait, les autres polyphénols extraits pris indépendamment les uns des autres étant présents sous forme de traces.

Parmi les 2 polyphénols majoritairement extraits selon l'invention, la quercitrine, qui est un glycoside de flavonol connu, n'a encore jamais été citée dans la composition de *Schinus molle,* et plus particulièrement des parties aériennes.

Parmi les autres polyphénols extraits, on retrouve ceux communément présents dans *Schinus molle* tels que par exemple l'isoquercitrine.

Par traces, on entend une concentration pour chaque polyphénol extrait pris indépendamment inférieure à 0,01% (100 ppm) en poids du poids total de l'extrait.

Les parties aériennes de *Schinus molle* désignent les feuillesen tant que telles et également les feuilles non débarassées des petites tiges portant les feuilles. Dans le cours de la description les termes « parties aériennes » et « feuilles non débarassées des petites tiges portant les feuilles », seront utilisées indiffféremment.

Les extraits de *Schinus molle* selon l'invention sont toutefois très difficilement caractérisables en tant que tels car leur composition varie, pour une même espèce, suivant différents facteurs tels que les parties de *Schinus molle* utilisées, le lieu de récolte, le mode de récolte, les conditions climatiques, ou encore l'année de récolte...

Avantageusement, le *Schinus molle* préférentiellement utilisé selon l'invention est cultivé dans des zones éloignées des pollutions urbaines, par exemple en altitude en Amérique du Sud, l'extrait obtenu selon l'invention ne comprenant pas d'éléments micropolluants tels que des phtalates, des pesticides, d'hydrocarbures aromatiques polycycliques ou de métaux lourds. Il est plus particulièrement récolté de juin à septembre.

L'extrait selon l'invention est obtenu à partir des feuilles non débarrassées des petites tiges portant les feuilles séchées et broyées de *Schinus molle* par extraction solide-liquide, sous agitation, de 5% à 10% en poids de matière solide par rapport au poids total mis en œuvre dans un solvant hydro-alcoolique, l'alcool étant choisi parmi l'éthanol ou le propanediol, dans une proportion comprise entre 60% et 80% en poids d'alcool par rapport au poids total du solvant, à une température comprise entre 20°C et 70°C pendant une durée comprise entre 1 heure et 2 heures, sous vide, à pression atmosphérique ou sous pression, avantageusement à pression atmosphérique, et par séparation des phases liquide et solide de manière à éliminer la phase solide et récupérer un extrait de *Schinus molle* liquide.

Par fraiche on entend la phase après ramassage pendant laquelle le végétal présente une humidité similaire à celle du végétal avant la cueillette.

Par séchage on entend la phase après ramassage au cours de laquelle le végétal est déshydraté jusqu'à une humidité dite de sauvegarde. Cette dessiccation a pour but de réduire suffisamment la teneur en eau notamment des feuilles non débarassées des petites tiges portant les feuilles pour garantir des conditions favorables de stockage ou de transformation ultérieure du végétal.

A titre d'exemples, les feuilles non débarassées des petites tiges portant les feuilles peuvent être séchées naturellement par exposition à l'air (au soleil ou à l'ombre) en contrôlant l'humidité relative pendant une durée d'au maximum 10 à 20 jours ou avantageusement délicatement séchées artificiellement avec de l'air soufflé par un séchoir, générateur d'air chaud ou ventilateur, à une température comprise entre 20°C et 40°C.

Préférentiellement, après avoir été avantageusement lavées, les feuilles non débarassées des petites tiges portant les feuilles sont séchées, utilisées entières ou broyées, préférentiellement broyées voire cryobroyées.

Après l'étape de ramassage, éventuellement lavage, et avantageusement séchage puis broyage des feuilles non débarassées des petites tiges portant les feuilles de *Schinus molle,* une étape de dispersion et d'extraction dans un solvant hydro-alcoolique est réalisée.

L'extraction s'effectue avantageusement toujours sous agitation permettant ainsi une dispersion et une homogénéisation du solide dans le liquide, améliorant efficacement la diffusion du soluté dans le solvant.

L'eau utilisée pour l'extraction est une eau distillée, déminéralisée ou encore une eau riche en sels minéraux et/ou oligo-éléments, préférentiellement une eau distillée.

Pour extraire majoritairement la quercitrine et la miquelianine d'intérêt parmi les polyphénols extraits de *Schinus molle,* un co-solvant alcoolique choisi parmi l'éthanol ou le propanediol, par exemple le 1,2-propanediol ou le 1,3-propanediol, préférentiellement le 1,3-propanediol, dans une proportion comprise entre 60% et 80% en poids d'alcool par rapport au poids total du solvant est utilisé.

A l'issue de l'extraction, la matière végétale, résiduelle et épuisée en composés d'intérêt, est avantageusement séparée de la phase liquide par exemple par décantation, par centrifugation ou par filtration clarifiante.

La phase liquide obtenue peut être microfiltrée à l'aide de médias filtrants de porosité adaptée afin d'obtenir une solution limpide et exempte de particules solides végétales.

Cette étape de séparation liquide-solide peut être avantageusement suivie d'une ou plusieurs étapes de purification par exemple par microfiltration, ultrafiltration et/ou nanofiltration, permettant de concentrer les composés d'intérêt au dépend d'autres composés extraits tels que des protéines et polysaccharides, ainsi que du solvant.

La durée et le mode d'extraction jouent ainsi un rôle essentiel sur les substances extraites, à savoir majoritairement la quercitrine et la miquelianine parmi les polyphénols extraits, les autres polyphénols extraits étant présents sous forme de traces.

L'extraction hydro-alcoolique pendant un temps nécessaire et suffisant permet avantageusement de solubiliser la quercitrine et la miquelianine en quantité efficace dans l'extrait pour leurs propriétés protectrices anti-âge.

Bien que les co-solvants alcooliques utilisés selon l'invention présentent un même pouvoir solvant, le co-solvant alcoolique préférentiellement utilisé est le propanediol, encore plus préférentiellement le 1,3-propanediol.

Selon un premier mode de réalisation avantageux de l'invention, de tels co-solvants alcooliques (propanediol) permettent d'obtenir un extrait liquide dans lequel le co-solvant est conservé. L'extrait obtenu est ainsi physiologiquement acceptable et directement utilisable pour des applications cosmétiques.

Selon un second mode de réalisation de l'invention, lorsque l'alcool est l'éthanol, l'extrait de *Schinus molle* liquide obtenu est préférentiellement séché par exemple par atomisation, lyophilisation ou zéodratation de manière à obtenir un extrait de *Schinus molle* solide, l'éthanol étant évaporé.

Le séchage peut être réalisé en présence d'un support tel que la maltodextrine.

A cet effet, selon un autre aspect, l'invention concerne un procédé d'obtention d'un extrait de *Schinus molle* selon l'invention, suivant les étapes telles que décrites ci-avant, avantageusement selon le premier ou le second mode de réalisation.

Préférentiellement, le procédé d'obtention d'un extrait de *Schinus molle* selon l'invention comprend les étapes suivantes selon lesquelles :

a) on recueille les feuilles non débarrassées des petites tiges portant les feuilles de *Schinus molle* ;

b) on disperse les feuilles non débarrassées des petites tiges portant les feuilles de *Schinus molle* recueillies, séchées et broyées dans une proportion de 5% à 10% en poids de matière solide par rapport au poids total mis en œuvre, dans un solvant hydro-alcoolique, l'alcool étant choisi parmi l'éthanol ou le propanediol, dans une proportion comprise entre 60% et 80% en poids d'alcool par rapport au poids total du solvant ;

c) on réalise une extraction solide-liquide, sous agitation, à une température comprise entre 20°C et 70°C pendant une durée comprise entre 1 heure et 2 heures, sous vide, à pression atmosphérique ou sous pression, préférentiellement à pression atmosphérique ;

d) on sépare par exemple par décantation, centrifugation ou filtration clarifiante les phases liquide et solide de manière à éliminer la phase solide et récupérer un extrait hydro-alcoolique de *Schinus molle* liquide comprenant majoritairement de la quercitrine et de la miquelianine parmi les polyphénols extraits, en une concentration respectivement d'au moins 0,04% de quercitrine en poids du poids total de l'extrait et au moins 0,02% de miquelianine en poids du poids total de l'extrait, les autres polyphénols extraits pris indépendamment les uns des autres étant présents sous forme de traces, c'est à dire à une concentration pour chaque polyphénol extrait pris indépendamment inférieure à 0,01% (100 ppm) en poids du poids total de l'extrait ; et

e) éventuellement, lorsque l'alcool est l'éthanol, on sèche par exemple par atomisation, lyophilisation ou zéodratation l'extrait de *Schinus molle* liquide obtenu de manière à obtenir un extrait de *Schinus molle* solide.

L'extrait de *Schinus molle* liquide ainsi obtenu peut être purifié par microfiltration, ultrafiltration et/ou nanofiltration pour concentrer l'extrait en quercitrine et miquelianine par rapport aux protéines et polysaccharides également extraits.

A titre illustratif, des exemples préférés de mode de réalisation de l'invention sont décrits ci-dessous.

Exemple 1 : Préparation d'un extrait hydro-glycolique à partir des parties aériennes de Schinus molle

Les feuilles non débarrassées des petites tiges portant les feuilles de *Schinus molle,* préalablement séchées, sont cryobroyées dans un broyeur à couteaux comportant une grille de 1 mm, ce qui permet d'obtenir une poudre dont la taille des particules se situe majoritairement vers 500 μm.

La poudre (150 g) ainsi obtenue est alors placée dans un réacteur en verre, muni d'une agitation et d'un chauffage par double enveloppe à l'aide d'un fluide caloporteur.

Le solvant utilisé (1350 g) pour la macération est un mélange de 70% de 1,3-propanediol (DuPont Tate & Lyle Bio Products Company) et 30% d'eau distillée.

La poudre de feuilles non débarrassées des petites tiges portant les feuilles est dispersée dans le solvant pendant 2 heures à 50°C puis le mélange est filtré.

L'extrait en milieu solvant ainsi obtenu, soit 945 g, est ensuite dosé par la méthode de Folin-Ciocalteu qui permet de doser l'ensemble des polyphénols. La méthode utilise l'acide gallique comme standard et les résultats sont exprimés en % massique équivalent acide gallique. Cet extrait contient donc 0,43% m/m équivalent acide gallique de polyphénols totaux, et plus particulièrement 0,1% m/m de quercitrine et 0,06% m/m de miquelianine pour un extrait sec de 2,4% m/m, soit 18% de polyphénols totaux, dont 4,2% de quercitrine et 2,5% de miquelianine dans l'extrait sec.

Exemple 2 : Préparation d'un extrait hydro-éthanolique à partir des parties aériennes de Schinus molle

Les feuilles non débarrassées des petites tiges portant les feuilles de *Schinus molle,* préalablement séchées, sont cryobroyées dans un broyeur à couteaux comportant une grille de 1 mm, ce qui permet d'obtenir une poudre dont la taille des particules se situe majoritairement vers 500 μm.

La poudre (150 g) ainsi obtenue est placée dans un réacteur en verre, muni d'une agitation et d'un chauffage par double enveloppe à l'aide d'un fluide caloporteur.

Le solvant utilisé (1350 g) pour la macération est de l'éthanol à 70% et 30% d'eau distillée.

La poudre de feuilles non débarrassées des petites tiges portant les feuilles est dispersée dans le solvant pendant 2 heures à 50°C puis le mélange est filtré.

L'extrait éthanolique est évaporé au rotavapor afin d'éliminer la majeure partie du solvant, puis lyophilisé afin d'obtenir un solide qui sera ensuite broyé.

Il est ainsi obtenu 13 g d'extrait sec de feuilles non débarrassées des petites tiges portant les feuilles de *Schinus molle,* dosé par la méthode de Folin-Ciocalteu et contenant 14% (équivalent acide gallique) de polyphénols totaux, et plus particulièrement 4% de quercitrine et 2% de miquelianine dans l'extrait sec.

Par comparaison, les Demandeurs ont réalisé d'autres modes d'extraction permettant l'obtention d'un extrait de *Schinus molle.*

Par exemple, un extrait éthanolique, obtenu par extraction dans l'éthanol pur, de feuilles non débarrassées des petites tiges portant les feuilles de *Schinus molle* réalisé dans des conditions comparables à celles de l'Exemple 2 ci-dessus permet d'obtenir un extrait sec contenant seulement 8% de polyphénols totaux, et plus particulièrement 3% de quercitrine et 1,2% de miquelianine dans l'extrait sec.

Le procédé selon l'invention, et notamment le choix du solvant hydro-alcoolique, permet de limiter sensiblement voire d'éviter d'extraire la fraction grasse extraite des feuilles de *Schinus molle* avec le solvant uniquement alcoolique (100%) et ainsi de concentrer les polyphénols extraits.

D'après ces résultats, on peut remarquer que le mode d'extraction, qui est facile à mettre en œuvre, est essentiel pour obtenir un extrait de *Schinus molle* comprenant majoritairement de la quercitrine et de la miquelianine parmi les polyphénols extraits, en une concentration respectivement d'au moins 0,04% de quercitrine en poids du poids total de l'extrait et au moins 0,02% de miquelianine, les autres polyphénols extraits pris indépendamment les uns des autres étant présents sous forme de traces, et qui soit ainsi utilisable en cosmétique.

Les polyphénols majoritairement extraits contenus dans l'extrait de *Schinus molle* selon l'invention sont plus particulièrement des agents actifs anti-âge qui agissent notamment pour améliorer la fonction barrière de la peau et protéger la peau contre les polluants atmosphériques.

En effet, les tissus en contact avec le milieu extérieur tels que la peau, les cheveux, les ongles et les poumons sont directement et constamment exposés aux polluants présents dans l'environnement et potentiellement nocifs.

Dans le cadre de l'invention, les polluants atmosphériques désignent les polluants présents dans l'environnement, qui sont néfastes pour la santé et se présentent notamment sous forme de gaz et de particules respirables. Ils peuvent être présents à la fois à l'extérieur, par exemple les particules de moteur diesel, l'ozone, ou les métaux lourds, et/ou à l'intérieur des maisons où la pollution peut notamment être due à la fumée de cigarette ou aux solvants libérés par les peintures, les colles ou les papiers peints, comme le toluène, styrène, xylène, benzaldéhyde.

Parmi les polluants présents dans l'environnement, on distingue les polluants primaires qui sont directement issus des sources de pollution (trafic routier, industries, chauffage, agriculture...) et les polluants secondaires qui proviennent de réactions chimiques de gaz entre eux. Parmi les polluants primaires, on peut par exemple citer les oxydes de carbone, les oxydes de soufre, les oxydes d'azote, les hydrocarbures légers, les composés organiques volatils (COV), les métaux (plomb, mercure, cadmium...), et les particules fines ou ultrafines, telles que les PM10 (de diamètre inférieur à 10 $\mu$m) et les PM2.5 (de diamètre inférieur à 2,5 $\mu$m) qui peuvent être particulièrement toxiques par leur capacité à pénétrer profondément dans le système respiratoire, cardiovasculaire et cutané, leur taille étant inférieure à la taille des pores de la peau humaine. Parmi les polluants secondaires on trouve des particules secondaires, l'ozone, les oxydes d'azote.

L'extraction selon l'invention permet d'obtenir un extrait enrichi en composés actifs anti-âge, limpide et exempt de particules solides végétales, avantageusement stable en termes d'aspect, de couleur et d'odeur, qui peut être utilisé dans la formulation de compositions cosmétiques.

Avantageusement, selon un troisième aspect de l'invention, l'extrait de *Schinus molle* est utilisé dans la composition d'un produit cosmétique comprenant, à titre d'agent actif protecteur, une quantité efficace d'un extrait de *Schinus molle* selon l'invention, et un excipient physiologiquement acceptable.

Par quantité efficace, on désigne la quantité minimum d'extrait selon l'invention qui est nécessaire pour obtenir l'activité notamment de fonction barrière et de protection de la peau contre les polluants atmosphériques, sans que cette quantité soit toxique.

Avantageusement, l'extrait de *Schinus molle* est présent dans la composition à une concentration de 0,001 à 1% en poids par rapport au poids total de la composition, préférentiellement de 0,01 à 1%.

Encore plus préférentiellement, la composition selon l'invention comprend majoritairement de la quercitrine et de la miquelianine, et ne comprend substantiellement pas d'autres polyphénols ou seulement sous forme de traces, parmi les polyphénols extraits,.

Un excipient physiologiquement acceptable désigne un véhicule adapté à une mise en contact avec les couches externes de la peau ou des muqueuses, c'est-à-dire qui ne présente pas de toxicité et ne provoque pas d'irritation, de réponse allergique indue ou encore de réaction d'intolérance.

Un excipient physiologiquement acceptable peut comprendre un ou plusieurs composés.

La composition utilisable selon l'invention pourra être appliquée par toute voie appropriée, notamment orale, ou topique externe, et la formulation des compositions sera adaptée par l'homme du métier.

Préférentiellement, les compositions selon l'invention se présentent sous une forme adaptée à l'application par voie topique. Ces compositions doivent donc contenir un milieu physiologiquement acceptable, c'est-à-dire compatible avec la peau et les phanères, sans risque d'inconfort lors de leur application et couvrent toutes les formes cosmétiques adaptées.

Par application topique, on désigne le fait d'appliquer ou d'étaler l'extrait de *Schinus molle* selon l'invention, et plus particulièrement une composition le contenant, à la surface de la peau ou d'une muqueuse.

La peau désigne plus particulièrement la peau du visage, notamment le contour des yeux et la bouche, le nez, le front, le cou, les mains, ainsi que la peau de l'ensemble du reste du corps.

Les compositions pour la mise en œuvre de l'invention pourront notamment se présenter sous forme d'une solution aqueuse, hydro-alcoolique ou huileuse, d'une émulsion huile-dans-eau, eau-dans-huile ou émulsions multiples ; elles peuvent aussi se présenter sous forme de suspensions, ou encore poudres, adaptées à une application sur la peau, les muqueuses, les lèvres et/ou les cheveux.

Ces compositions peuvent être plus ou moins fluides et avoir également l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol.

A titre d'excipient physiologiquement acceptable communément utilisé dans le domaine d'application envisagé, on peut citer par exemple des adjuvants nécessaires à la formulation, tels que des solvants, des épaississants, des diluants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20% du poids total de la composition. Lorsque la composition selon l'invention est une émulsion, la phase grasse peut représenter de 5 à 80% en poids et de préférence de 5 à 50% en poids par rapport au poids total de la composition. Les émulsionnants et co-émulsionnants utilisés dans la composition sont choisis parmi ceux classiquement utilisés dans le domaine considéré. Par exemple, ils peuvent être utilisés en une proportion allant de 0,3 à 30% en poids, par rapport au poids total de la composition.

Selon un autre mode de réalisation avantageux de l'invention, l'extrait de *Schinus molle* selon l'invention peut être encapsulé ou inclus dans un vecteur cosmétique tels que les liposomes ou toute autre nanocapsule ou microcapsule utilisée dans le domaine de la cosmétique ou adsorbé sur des polymères organiques poudreux, des supports minéraux comme les talcs et bentonites.

Avantageusement, la composition selon l'invention peut comprendre, outre l'agent actif selon l'invention, au moins un autre agent actif présentant des effets cosmétiques similaires et/ou complémentaires à ceux de l'invention. Selon l'invention, cet agent actif est défini comme un « agent actif additionnel ».

Par exemple, le ou les agents actifs additionnels peuvent être choisis parmi : les agents anti-âge, raffermissants, éclaircissants, hydratants, drainants, favorisant la microcirculation, agents pharmaceutiques, exfoliants, desquamants, stimulant la matrice extracellulaire, activant le métabolisme énergétique, antibactériens, antifongiques, apaisants, anti-radicalaires, anti-UV, anti-acné, anti-inflammatoires, anesthésiques, procurant une sensation de chaleur, procurant une sensation de fraicheur, amincissants.

De tels agents additionnels peuvent être choisis dans les groupes comprenant :

- la vitamine A et notamment l'acide rétinoïque, le rétinol, le rétinol proprionate, le rétinol palmitate ;
- la vitamine B3 et plus particulièrement le niacinamide, le nicotinate de tocophérol ;
- la vitamine B5, la vitamine B6, la vitamine B12, le panthénol ;
- la vitamine C, notamment l'acide ascorbique, l'ascorbyl glucoside, l'ascorbyl tetrapalmitate, magnésium et sodium ascorbyl phosphate ;
- les vitamines E, F, H, K, PP, le coenzyme Q10 ;
- les inhibiteurs de métalloprotéinase, ou un activateur des TIMP ;
- la DHEA, ses précurseurs et dérivés ;
- les acides aminés tels que l'arginine, ornithine, hydroxyproline, hydroxyproline dipalmitate, palmitoylglycine, hydroxylysine, méthionine et ses dérivés, composés acides aminés N-acyl ;
- les peptides naturels ou de synthèse, incluant les di-, tri-, tetra-, penta- et hexapeptides et leurs dérivés lipophiles, isomères et complexés avec d'autres espèces telles qu'un ion métal (e.g. cuivre, zinc, manganèse, magnésium, et autres). A titre d'exemples, on peut citer les peptides commercialement connus sous le nom MATRIXYL®, ARGIRELINE®, CHRONOGEN™, LAMINIXYL IS™, PEPTIDE Q10™, COLLAXYL™ (brevet FR2827170, ASHLAND®), PEPTIDE VINCI 01™ (brevet FR2837098, ASHLAND®), PEPTIDE VINCI 02™ (brevet FR2841781, ASHLAND®), ATPeptide™ (brevet FR2846883, ASHLAND®) ou encore le peptide de synthèse de séquence Arg-Gly-Ser-NH$_2$, commercialisé sous le nom ATPeptide™ par ASHLAND® ;
- l'extrait d'*Artémia salina,* commercialisé sous le nom GP4G™ (FR2817748, ASHLAND®) ;
- les extraits peptidiques végétaux tels que les extraits de lin (Lipigénine™, brevet FR2956818, ASHLAND®), les extraits de soja, d'épeautre, de vigne, de colza, de lin, de riz, de maïs, de pois ;

- les extraits de levures, par exemple le Dynagen™, (brevet FR2951946, ASHLAND®) ou l'Actopontine™ (brevet FR2944526, ASHLAND®) ;
- l'acide déhydroacétique (DHA) ;
- les phystostérols d'origine synthétique ou naturelle ;
- l'acide salicylique et ses dérivés, les alpha- et bêta-hydroxyacides, les silanols ;
- les sucres aminés, glucosamine, D-glucosamine, N-acetyl glucosamine, N-acetyl-D-glucosamine, mannosamine, N-acetyl mannosamine, galactosamine, N-acetyl galactosamine ;
- les extraits de polyphénols, isoflavones, flavonoïdes, tels que les extraits de raisin, les extraits de pin, les extraits d'olives ;
- les lipides tels que les céramides ou les phospholipides, les huiles d'origine animale, telles que le squalène ou le squalane ; les huiles végétales, telles que l'huile d'amande douce, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, le beurre de karité ;
- tous écrans UV et filtres solaires ;
- l'AMP cyclique et ses dérivés, les agents activateurs de l'enzyme adénylate cyclase et les agents inhibiteurs de l'enzyme phosphodiestérase, l'extrait de *centella asiatica,* l'asiaticoside et l'acide asiatique, les méthyls xanthines, la théine, la caféine et ses dérivés, la théophylline, la théobromine, la forskoline, l'esculine et l'esculoside, les inhibiteurs d'ACE, le peptide Val-Trp, l'inhibiteurs du neuropeptide Y, l'enkephaline, l'extrait de *gingko biloba,* l'extrait de *dioscorea,* la rutine, l'extrait de yerba mate, l'extrait de guarana, les oligosaccharides, les polysaccharides, la carnitine, l'extrait de lierre, l'extrait de fucus, l'extrait hydrolysé de *Prunella vulgaris,* l'extrait hydrolysé de *Celosia cristata,* l'extrait d'*Anogeissus leiocarpus,* l'extrait de feuilles de *Manihot utilissima,* la palmitoylcarnitine, la carnosine, la taurine, l'extrait de sureau, les extraits d'algue tel que l'extrait de *Palmaria Palmata.*

A titre d'illustration, il est mentionné ci-après un exemple de formulation d'une composition cosmétique contenant un extrait de *Schinus molle* obtenu selon l'invention :

Exemple 3 : Crème de soin (Formule référence #TCAAE08)

| Ingrédients (Nom de marque) | INCI | % massique |
|---|---|---|
| **Phase A** | | |
| Eau purifiée | Aqua | QSP 100 |
| Na4 EDTA | Tetrasodium EDTA | 0,05 |
| Lubrasil™ II DM hydrogel | Glycerin (and) Glyceryl Acrylate/Acrylic Acid Copolymer (and) Laureth-23 (and) Dimethicone | 3,00 |
| Liquapar/Rokonsal™ MEP preservative | Phenoxyethanol (and) Methylparaben (and) Ethylparaben (and) Propylparaben | 1,00 |
| **Phase B** | | |
| UltraThix™ P-100 polymer | Acrylic Acid/VP Crosspolymer | 0,60 |
| **Phase C** | | |
| Hydroxide de Sodium | Sodium Hydroxyde | 0,02 |
| Eau purifiée | Aqua | 0,50 |
| **PhaseD** | | |
| Belsil* W3230 | Bis-Stearoxydimethylsilane, stearyl alcohol, Dimethicone | 2,00 |
| Simulsol* 165 | PEG-100 Stearate (and) Glyceryl Stearate | 2,00 |
| Beurre de Karité raffinér | Butyrospermum Parkii (Shea) Butter | 2,00 |
| Ceraphyl™ 28 ester | Cetyl Lactate | 1,50 |

(suite)

| PhaseD | | |
|---|---|---|
| Ceraphyl™ 791 ester | Isocetyl Stearoyl Stearate | 2,00 |
| Ceraphyl™ ODS ester | Octyldodecyl Stearate | 3,00 |
| Ceraphyl™ 368 ester | Ethylhexyl Palmitate | 4,00 |
| **Phase E** | | |
| Hydroxide de Sodium | Sodium Hydroxide | 0,03 |
| Eau purifiée | Aqua | 0,50 |
| **Phase F** | | |
| Extrait de *Schinus molle* selon l'exemple 1 | | 1,00 |

Procédé de préparation de la composition de l'exemple 3:

1. Ajouter la phase A dans le récipient principal et commencer à homogénéiser. Chauffer à 70-75°C.

2. Saupoudrer dans un UltraThix™ P-100 et bien mélanger pendant environ 30 minutes.

3. Ajouter les ingrédients de la phase D dans un bécher à part et chauffer à 70-75°C.

4. Ajouter la phase C pré-mélangée dans la phase A et mélanger jusqu'à homogénéité.

5. Maintenir à 70-75°C et ajouter la phase D dans le récipient principal et bien mélanger. L'émulsion doit être homogène.

6. Commencer le refroidissement.

7. A environ 50°C, ajouter la phase E pré-mélangée et bien mélanger.

8. A température ambiante, ajouter la phase F et mélanger jusqu'à uniformité. Arrêter à 25°C.

La composition selon l'exemple 3 se présente ainsi sous forme d'une crème blanche, stable, avec un pH compris entre 5,20 et 5,80 et une viscosité (D0) de 25000-45000 (Brookfield RVT/Spindle B/5 RPM/1 minute/25°C).
Une composition dite « placebo » est réalisée exactement de la même manière avec l'exception que l'ingrédient « Extrait de *Schinus molle* selon l'exemple 1 » est remplacé par de l'eau purifiée. Cette composition « placebo » est plus particulièrement utilisée à titre de contrôle dans certains tests d'activité ci-après.
Selon un autre aspect, l'invention concerne l'utilisation cosmétique d'une composition comprenant notamment une quantité efficace d'un extrait de *Schinus molle* obtenu selon l'invention, pour améliorer la fonction barrière de la peau et protéger contre les polluants atmosphériques.
La peau est un organe dont la partie supérieure, le *stratum corneum* ainsi que le film hydrolipidique qui le recouvre, présente un rôle de barrière physique protectrice vis-à-vis de l'environnement extérieur. Ce rôle de barrière plus communément nommé « fonction barrière de la peau » est d'une importance majeure dans l'homéostasie tissulaire et plus particulièrement dans la protection vis-à-vis d'agents extérieurs d'ordre pathogènes ou non, tels que les polluants atmosphériques environnementaux.
Or, on connait que des altérations de la fonction barrière résultent des stress extérieurs tels que les UV. Les conséquences sont majeures au niveau de la perte de la cohésion cellulaire et de l'intégrité mécanique. Certaines enzymes impliquées dans les phases terminales de la différenciation kératinocytaire jouent un rôle clé dans la protection face aux UV. Des modifications de l'expression et/ou de l'activité de ces enzymes ont des conséquences importantes sur l'intégrité de la fonction barrière et l'homéostasie de la peau.
Des altérations de la fonction barrière sont également observées dans des cas de peaux pathologiques et dans des cas de peaux saines âgées dues à des anomalies au niveau de la synthèse lipidique créant le plus souvent un manque de cohésion des cellules des couches superficielles de l'épiderme pouvant entrainer une augmentation de la perméabilité cutanée. Ainsi, par exemple, le manque de cohésion des cellules de l'épiderme qui se manifeste lors du vieillissement cutané, peut avoir pour conséquence des micro-fractures anguleuses et un élargissement des pores de la peau.

Des altérations de la fonction barrière peuvent être dues à des stress chimiques. Dans cette dernière catégorie, il faut prendre en considération les polluants atmosphériques environnementaux et plus particulièrement les microparticules PM2.5 produits par les moteurs diesel, la combustion du charbon, la fumée de cigarette. Ces microparticules de petites tailles sont en effet responsables de la majorité des effets négatifs imputés à la pollution dus à leur capacité à pénétrer profondément (Pan T-L et al. The impact of urban particulate pollution on skin barrier function and the subsequent drug absorption. J Dermatol Sci,2015).

En outre, certains de ces polluants atmosphériques environnementaux ont des propriétés irritantes pour la peau ou peuvent provoquer des réactions inflammatoires. Or l'inflammation pourrait être responsable d'une sécrétion accrue de sébum (Lefebvre M.A. et al. « Evaluation of the impact of urban pollution on the quality of skin: a multicentre study in Mexico,Int J Cosmet Sci. 2015 Feb 6).

Au quotidien, la peau est ainsi régulièrement soumise à des agressions environnementales (UV, pollution...) qui peuvent altérer le fonctionnement physiologique de cet organe barrière (Pan T-L et al. The impact of urban particulate pollution on skin barrier function and the subsequent drug absorption. J Dermatol Sci,2015).

La composition selon l'invention comprenant une quantité efficace d'un extrait de *Schinus molle* permet de lutter contre ces agressions extérieures en favorisant une différenciation et une cohésion optimale des couches cutanés permettant de maintenir une structure histologique fonctionnelle et une perméabilité d'ordre physiologique nécessaire pour maintenir cette fonction essentielle de barrière que représente la peau au niveau du tissu et au-delà, au niveau de l'organisme dans son ensemble.

La composition selon l'invention participe ainsi à l'hydratation de la peau et à la lutte contre l'apparition des signes du vieillissement cutané.

Par « signes du vieillissement cutané » on entend toutes modifications de l'aspect extérieur de la peau dues au vieillissement comme, par exemple, les rides et ridules, les crevasses, les poches sous les yeux, les cernes, le flétrissement, la perte d'élasticité et/ou de tonus de la peau, le ternissement ou le manque d'éclat, les défauts pigmentaires mais également toutes modifications internes de la peau qui ne se traduisent pas systématiquement par un aspect extérieur modifié comme, par exemple, l'amincissement de la peau, ou toutes dégradations internes de la peau consécutives à une exposition aux rayonnements UV.

La composition selon l'invention est ainsi également utilisée pour limiter la sécrétion de sébum et réduire la taille des pores de la peau.

A ce titre, l'invention est illustrée ci-après, par les différents résultats de tests réalisés.

Exemple 4 : Effets d'un extrait hydro-éthanolique de *Schinus Molle* obtenu selon l'exemple 2 sur l'expression de marqueurs de la différenciation, de l'hydratation et de la synthèse des lipides dans des kératinocytes (modèles *in vitro)*

Les effets potentiels d'un extrait hydro-éthanolique de *Schinus Molle* obtenu selon l'exemple 2 sur l'hydratation cutanée et la fonction barrière ont été recherchés dans un modèle de kératinocytes épidermiques humains normaux (NHEK), en analysant le profil d'expression de gènes spécifiques de la différenciation des kératinocytes et de la synthèse des lipides par une méthode de RT-qPCR (Reverse transcription quantitative polymerase chain reaction).

Modèles biologiques :

Kératinocytes épidermiques humain normaux (NHEK)

| | |
|---|---|
| Type cellulaire: | NHEK, utilisés au 3e passage |
| Conditions de culture : | 37°C, 5% $CO_2$ |
| Milieu de culture: | KératinocyteSFM (Serum free medium) complémenté avec Epidermal Growth Factor (EGF) 0,25 ng/ml Extrait Pituitaire (EP) 25 $\mu$g/ml Gentamycine 25 $\mu$g/ml |
| Milieu d'essai: | KératinocyteSFM complémenté avec Gentamycine 25 $\mu$g/ml |

Composés testés

Extrait hydro-éthanolique de *Schinus molle* obtenu selon l'exemple 2, sous forme de poudre, stocké à température ambiante ;
Solution stock à 10% (100 mg/ml) en DMSO (Dimethyl sulfoxide) ;
Concentrations testées : 0,0001% ; 0,0003% et 0,001% sur NHEK.

Culture et traitement

Les kératinocytes épidermiques humains normaux (NHEK) ont été ensemencés et cultivés en milieu de culture pendant 24 heures. Le milieu a ensuite été remplacé par le milieu d'essai contenant ou non (témoin) l'extrait hydro-éthanolique de *Schinus molle* obtenu selon l'exemple 2 testé ou la référence positive de différenciation (chlorure de calcium à 1,5 mM) et les cellules ont été incubées pendant 24 heures. Toutes les conditions ont été réalisées en n=3.
A la fin de l'incubation, les surnageants de culture ont été éliminés et les tapis cellulaires ont été rincés avec une solution de PBS (Phosphate buffered saline). Les plaques ont été immédiatement congelées à sec à -80°C.

Analyse de l'expression différentielle des marqueurs par PCR array :

L'expression des marqueurs a été évaluée par RT-qPCR sur les ARNm (ARN messagers) extraits des tapis cellulaires de chaque traitement (les réplicats ont été poolés avant l'extraction de l'ARN).
L'analyse de l'expression des gènes a été réalisée en n=2 à l'aide de 2 « Marker quantitative PCR array » contenant 16 gènes dont 2 gènes de ménage HK (Housekeeping), dédiés à la recherche et adaptés au format « screening » (mQPA-NHEK-BARRIER-16, produit par BIOalternatives®).

Transcription inverse

Les ARN totaux de chaque échantillon ont été extraits à l'aide de TriPure Isolation Reagent® selon le protocole préconisé par le fournisseur. La quantité et la qualité des ARN ont été évaluées par électrophorèse capillaire (Bioanalyzer 2100, Agilent).
Les traces d'ADN potentiellement contaminant ont été éliminées par traitement avec le système DNA-free (Ambion). Les ADNc (Acides desoxyribonucléiques complémentaires) ont été synthétisés par transcription inverse des ARN (Acides ribonucléiques) totaux en présence d'oligo(dT) et de l'enzyme « Transcriptor Reverse Transcriptase » (Roche®). L'ADNc obtenu a été quantifié par spectrophotométrie (Nanovue ; GE Healthcare®), puis les quantités d'ADNc ont été ajustées.

PCR Quantitative

Les réactions de PCR (polymerase chain reactions) ont été réalisées par PCR quantitative (Light Cycler ; Roche Molecular Systems Inc.) et selon les procédures recommandées par le fournisseur.
Le mélange réactionnel (10 $\mu$l final) pour chaque échantillon contenait :

2,5 $\mu$l d'ADNc,
les amorces des différents marqueurs utilisés,
le mélange réactionnel contenant l'enzyme taq DNA polymérase, et
le marqueur SYBR Green I et du $MgCl_2$.

Les données brutes de PCR quantitative ont été transférées et traitées sous le logiciel Microsoft Excel®.
L'incorporation de fluorescence dans l'ADN amplifié est mesurée en continu au cours des cycles de PCR. Ces mesures permettent d'obtenir des courbes d'intensité de la fluorescence en fonction des cycles de PCR et d'évaluer ainsi une valeur d'expression relative (ER) pour chaque marqueur.
Le nombre de cycles est déterminé à partir des points de « sortie » des courbes de fluorescence. Pour un même marqueur analysé, plus un échantillon sort tard (apparait tardivement, après un nombre de cycles élevé), plus le nombre initial de copies de l'ARNm est faible.
La valeur d'ER (expression relative) est exprimée en unités arbitraires (UA) selon la formule suivante :

EP 3 280 497 B1

$$(1/2^{\text{nombre de cycles}}) \times 10^6$$

Les PCR array « mQPA-NHEK-BARRIER-16 » contiennent 2 gènes de références (gènes de ménage ou « housekeeping ») utilisés pour la normalisation des données. Ces 2 gènes de ménage, les gènes RPS28 (Ribosomal protein S28) et GAPDH (Glyceraldehyde-3-phosphate dehydrogenase), sont exprimés de façon constitutive et leur niveau d'expression n'est pas ou peu impacté par les traitements. Ainsi, le niveau d'expression de chaque marqueur est comparé à la moyenne d'expression de ces 2 gènes de référence.

Traitement des données

Les données brutes ont été transférées et traitées sous le logiciel Microsoft Excel®.

Formules utilisées dans ce rapport :

Erreur standard de la moyenne : esm = écart-type (Sd)/√n
L'erreur standard de la moyenne (esm) représente l'écart de la moyenne de l'échantillon par rapport à la moyenne de la vraie population. L'esm est calculée en divisant l'écart-type (Sd) par la racine carrée de la taille de l'échantillon.

Résultats

Les résultats sont présentés dans les Tableaux 1 et 2 ci-dessous.

- "CaCl$_2$" résultats complets (nombre de cycles et pourcentage du témoin) et effets de la référence (CaCl$_2$) sur l'expression des différents marqueurs étudiés triés par « cluster » ;

- "extrait hydro-éthanolique de *Schinus molle* obtenu selon l'exemple 2": résultats complets (nombre de cycles et pourcentage du témoin) et effets de ce composé sur l'expression des gènes, triés par « cluster ».

14

Tableaux 1 et 2: Effets d'un extrait hydro-éthanolique de *Schinus molle* selon l'exemple 2 sur l'expression de marqueurs de la différenciation, de l'hydratation et de la synthèse des lipides dans des kératinocytes évalués par une méthode de RT-qPCR

| mQPA-NHEK-BARRIER-16 | Gènes | | Témoin | Tableau 1 : Extrait hydro-éthanolique de *Schinus molle* obtenu selon l'exemple 2 | | | | | |
|---|---|---|---|---|---|---|---|---|---|
| | | | | 0,0001% | | 0,0003% | | 0,001% | |
| | | | Cycles | Cycles | % Témoin (Moyenne HK) | Cycles | % Témoin (Moyenne HK) | Cycles | % Témoin (Moyenne HK) |
| | Nom complet et Abréviation | | | | 100 | | 100 | | 100 |
| Gènes de ménage (HK) | Ribosomal protein S28 | RPS28 | 18,79 | 19,29 | 86 | 19,34 | 89 | 19,52 | 80 |
| | | | 18,81 | 19,29 | | 19,22 | | 19,50 | |
| | Glyceraldeh yde-3-phosphate dehydrogenase | GAPDH | 16,77 | 17,02 | 103 | 17,10 | 103 | 17,22 | 105 |
| | | | 16,91 | 17,12 | | 17,10 | | 17,11 | |
| Hydratation, transport de solutés | Aquaporin 3 (Gill blood group) | AQP3 | 22,95 | 23,75 | 73 | 23,24 | 111 | 22,66 | 172 |
| | | | 23,06 | 23,73 | | 23,08 | | 22,58 | |
| Matrice extracellulaire | Hyaluronan synthase 3 | HAS3 | 25,15 | 25,82 | 78 | 26,43 | 59 | 26,50 | 54 |
| | | | 25,28 | 25,87 | | 26,15 | | 26,52 | |
| | Acyl-CoA synthetase short-chain family member 2 | ACSS2 | 23,42 | 23,78 | 100 | 23,83 | 101 | 23,85 | 106 |
| | | | 23,57 | 23,78 | | 23,73 | | 23,76 | |
| | Fatty acid synthase | FASN | 21,30 | 22,00 | 77 | 21,98 | 80 | 22,25 | 69 |
| | | | 21,32 | 21,93 | | 21,88 | | 22,22 | |
| | Serine palmitoyltransferase, long chain base subunit 1 | SPTLC1 | 24,19 | 24,65 | 85 | 24,10 | 129 | 24,29 | 115 |
| | | | 24,15 | 24,71 | | 24,11 | | 24,45 | |
| | LAG1 homolog, ceramide synthase 6 | LASS6 | 27,52 | 27,68 | 110 | 27,70 | 108 | 27,25 | 157 |
| | | | 27,65 | 27,77 | | 27,87 | | 27,41 | |

(suite)

| mQPA-NHEK-BARRIER-16 | Gènes | | Témoin | Tableau 1 : Extrait hydro-éthanolique de *Schinus molle* obtenu selon l'exemple 2 | | | | | |
|---|---|---|---|---|---|---|---|---|---|
| | | | | 0,0001% | | 0,0003% | | 0,001% | |
| | | | Cycles | Cycles | % Témoin (Moyenne HK) | Cycles | % Témoin (Moyenne HK) | Cycles | % Témoin (Moyenne HK) |
| | Nom complet et Abréviation | | | | 100 | | 100 | | 100 |
| **Synthèse des lipides** | UDP-glucose ceramide glucosyltra nsferase | **UGCG** | 25,91 | 25,71 | 150 | 26,19 | 112 | 25,96 | 133 |
| | | | 25,93 | 25,52 | | 25,93 | | 25,86 | |
| | Sphingomyel in phosphodies terase 1, acid lysosomal | **SMPD1** | 26,81 | 28,34 | 40 | 28,57 | 36 | 28,15 | 53 |
| | | | 26,89 | 28,60 | | 28,66 | | 28,19 | |
| | Glucosidase, beta; acid (includes glucosylcer amidase) | **GBA** | 24,97 | 25,18 | 110 | 25,33 | 97 | 25,43 | 94 |
| | | | 25,09 | 25,15 | | 25,41 | | 25,62 | |
| | Sulfotransferase family, cytosolic, 2B, member 1 | **SULT2B1** | 25,42 | 26,59 | 59 | 25,72 | 102 | 25,08 | 160 |
| | | | 25,52 | 26,43 | | 25,77 | | 25,30 | |
| | Arachidonate 12-lipoxygenase | **ALOX12** | 27,73 | 28,93 | 50 | 29,31 | 45 | 28,59 | 64 |
| | | | 27,57 | 28,92 | | 28,94 | | 28,79 | |
| **Différencia tion des kératinocytes** | Claudin 1 | **CLDN1** | 22,78 | 23,50 | 75 | 22,71 | 122 | 22,21 | 195 |
| | | | 22,84 | 23,50 | | 22,96 | | 22,28 | |
| | Filaggrin | **FLG** | 25,12 | 25,51 | 95 | 25,21 | 113 | 24,94 | 158 |
| | | | 24,98 | 25,29 | | 25,14 | | 24,64 | |
| | Involucrin | **IVL** | 24,86 | 25,70 | 67 | 25,13 | 94 | 25,54 | 87 |
| | | | 24,95 | 25,82 | | 25,47 | | 25,47 | |
| | Transglutaminase 1 (K polypeptide epidermal type I, protein-glutamine-gamma-glutamyl transferase) | **TGM1** | 28,06 | 29,03 | 74 | 28,29 | 119 | 27,81 | 182 |
| | | | 28,28 | 28,74 | | 28,15 | | 27,60 | |

| mQPA-NHEK-BARRIER-16 | Gènes | | Témoin | Tableau 2 : CaCl$_2$ - 1,5 mM | | | |
|---|---|---|---|---|---|---|---|
| | | | Cycles | Cycles | % Témoin (RPS28) | %Témoin (GAPDH) | % Témoin (Moyenne HK) |
| | Nom complet et Abréviation | | | | | | 100 |
| Gènes de ménage (HK) | Ribosomal protein S28 | RPS28 | 18,79 | 19,13 | 100 | 96 | 97 |
| | | | 18,81 | 19,13 | | | |
| | Glyceraldehyde-3-phosphate dehydrogenase | GAPDH | 16,77 | 17,16 | 104 | 100 | 101 |
| | | | 16,91 | 17,06 | | | |
| Hydratation, transport de solutés | Aquaporin 3 (Gill blood group) | AQP3 | 22,95 | 23,18 | 115 | 110 | 111 |
| | | | 23,06 | 23,09 | | | |
| Matrice extracellulaire | Hyaluronan synthase 3 | HAS3 | 25,15 | 25,34 | 112 | 108 | 108 |
| | | | 25,28 | 25,42 | | | |

EP 3 280 497 B1

(suite)

| mQPA-NHEK-BARRIER-16 | Gènes | | Témoin | Tableau 2 : CaCl$_2$ - 1,5 mM | | | |
|---|---|---|---|---|---|---|---|
| | | | Cycles | Cycles | % Témoin (RPS28) | %Témoin (GAPDH) | % Témoin (Moyenne HK) |
| | Nom complet et Abréviation | | | | | | 100 |
| Synthèse des lipides | Acyl-CoA synthetase short-chain family member 2 | ACSS2 | 23,42 / 23,57 | 23,34 / 23,51 | 132 | 127 | 128 |
| | Fatty acid synthase | FASN | 21,30 / 21,32 | 21,51 / 21,51 | 109 | 105 | 106 |
| | Serine palmitoyltransferase, long chain base subunit 1 | SPTLC1 | 24,19 / 24,15 | 24,58 / 24,46 | 99 | 95 | 96 |
| | LAG1 homolog, ceramide synthase 6 | LASS6 | 27,52 / 27,65 | 27,80 / 27,83 | 107 | 103 | 104 |
| | UDP-glucose ceramide glucosyltransferase | UGCG | 25,91 / 25,93 | 25,96 / 25,94 | 123 | 118 | 119 |
| | Sphingomyelin phosphodiesterase 1, acid lysosomal | SMPD1 | 26,81 / 26,89 | 27,01 / 27,00 | 113 | 108 | 109 |
| | Glucosidase, beta; acid (includes glucosylceramidase) | GBA | 24,97 / 25,09 | 24,96 / 25,04 | 128 | 123 | 124 |
| | Sulfotransferase family, cytosolic, 2B, member 1 | SULT2B1 | 25,42 / 25,52 | 24,44 / 24,47 | 254 | 244 | 246 |
| | Arachidonate 12-lipoxygenase | ALOX12 | 27,73 / 27,57 | 27,18 / 27,48 | 158 | 151 | 152 |

EP 3 280 497 B1

(suite)

| mQPA-NHEK-BARRIER-16 | Gènes | | Témoin | Tableau 2 : $CaCl_2$ - 1,5 mM | | | |
|---|---|---|---|---|---|---|---|
| | | | **Cycles** | **Cycles** | **% Témoin (RPS28)** | **%Témoin (GAPDH)** | **% Témoin (Moyenne HK)** |
| | Nom complet et Abréviation | | | | | | 100 |
| **Différenciation des kératinocytes** | Claudin 1 | **CLDN1** | 22,78 | 22,55 | 152 | 145 | 147 |
| | | | 22,84 | 22,53 | | | |
| | Filaggrin | **FLG** | 25,12 | 24,79 | 157 | 151 | 152 |
| | | | 24,98 | 24,66 | | | |
| | Involucrin | **IVL** | 24,86 | 24,42 | 183 | 175 | 177 |
| | | | 24,95 | 24,31 | | | |
| | Transglutaminase 1 (K polypeptide epidermal type I, protein-glutamine-gamma-glutamyltransferase) | **TGM1** | 28,06 | 27,01 | 285 | 274 | 276 |
| | | | 28,28 | 26,96 | | | |

Dans les Tableaux 1 et 2, on considère qu'il y a stimulation, lorsque le % est supérieur ou égal à 150. Au contraire, on considère qu'il y a inhibition, lorsque le % est inférieur ou égal à 65%.

L'incubation des NHEK avec la référence positive de différenciation CaCl$_2$ à 1,5 mM, a augmenté l'expression des ARNm codant pour des marqueurs de la synthèse des lipides (SULT2B1 et ALOX12) et de la différenciation des kératinocytes (FLG, IVL et TGM1). Ces résultats étaient attendus et ont permis de valider l'essai.

L'extrait hydro-éthanolique de *Schinus molle* obtenu selon l'exemple 2, à la plus forte concentration testée (0,001%), a stimulé l'expression des marqueurs de différenciation des kératinocytes CLDN1, FLG et TGM1. De plus, une augmentation de l'expression du marqueur de l'hydratation AQP3 et des marqueurs de la synthèse des lipides LASS6 et SULT2B1 a également été observée. Ceci a été associé à une baisse de l'expression de certains gènes impliqués dans la synthèse des lipides (SMPD1 et ALOX12). Aux deux plus faibles concentrations testées (0,0001% et 0,0003%), seule l'inhibition de l'expression de SMPD1 et ALOX12 a été observée.

Conclusion

Dans les conditions expérimentales de cette étude, les résultats obtenus suggèrent que l'extrait hydro-éthanolique de *Schinus molle* obtenu selon l'exemple 2, à la plus forte concentration testée (0,001%), aurait un effet positif sur l'hydratation et la fonction barrière de l'épiderme.

Exemple 5 : Evaluation de l'effet protecteur de l'extrait de Schinus molle selon l'exemple 1 sur des kératinocytes soumis à un stress environnemental par des particules polluantes PM10-like.

Le but de cette étude est de déterminer l'effet protecteur de l'extrait de *Schinus molle* selon l'exemple 1 à 0,1%, sur des kératinocytes soumis à un stress environnemental. La pollution de l'air est associée à la présence de fine particules ayant une taille inférieure à 10 $\mu$M (PM10-like).

La mesure du stress cellulaire est réalisée par le dosage de l'activité enzymatique de la lactacte déshydrogénase (LDH) libérée dans le milieu extracellulaire par les cellules. La LDH est une enzyme oxydoréductase qui catalyse la conversion du pyruvate en lactate. Les cellules libèrent cette enzyme dans le milieu extracellulaire lorsqu'il y a une atteinte de l'intégrité cellulaire.

Ce test enzymatique a été largement utilisé pour évaluer la présence de lésions et de toxicité des tissus et cellules.

Protocole:

Les kératinocytes humains normaux sont cultivés dans du milieu sans sérum puis traités avec un extrait de *Schinus molle* selon l'exemple 1 à 0,1% dilué dans le milieu de culture pendant 48h à raison de 2 applications par jour. Le contrôle non traité est réalisé dans les mêmes conditions. Au bout de 48h, des particules polluantes PM10-like sont appliquées sur les cellules à deux concentrations 250 $\mu$g/ml ou 500 $\mu$g/ml pendant 24h avec une seule application.

Le lendemain, le milieu extracellulaire est prélevé afin de mesurer l'activité enzymatique de la lactate déshydrogénase en utilisant le protocole précis et détaillé du kit Lactate Dehydrogenase Activity Assay Kit (Sigma-aldrich, MAK066). Chaque condition est réalisée en triplicat.

Résultats:

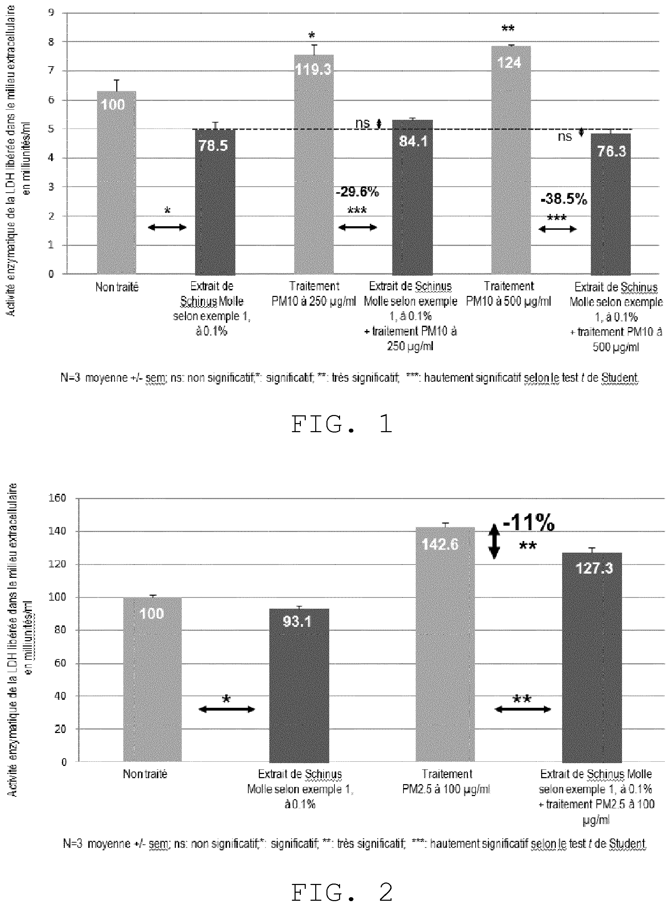

Les résultats présentés dans la Figure 1 obtenus *in vitro* montrent que l'extrait de *Schinus molle* selon l'exemple 1 à 0,1% protège, de manière hautement significative, les kératinocytes soumis à un stress aux particules PM10-like aux doses de 250 $\mu$g/ml (-29,6% de LDH libérée comparé au traitement PM10-like 250 $\mu$g/ml) ou 500 $\mu$g/ml (-38,5% de LDH libérée comparé au traitement PM10-like 500 $\mu$g/ml).

Exemple 6 : Evaluation de l'effet protecteur de l'extrait de Schinus molle selon l'exemple 1 sur des kératinocytes soumis à un stress environnemental par des particules polluantes PM2.5-like.

Le but de cette étude est de déterminer l'effet protecteur de l'extrait de *Schinus molle* selon l'exemple 1 à 0,1%, sur des kératinocytes soumis à un stress environnemental.

La mesure du stress cellulaire est réalisée par le dosage de l'activité enzymatique de la lactacte déshydrogénase (LDH) libérée dans le milieu extracellulaire par les cellules. La LDH est une enzyme oxydoréductase qui catalyse la conversion du pyruvate en lactate. Les cellules libèrent cette enzyme dans le milieu extracellulaire lorsqu'il y a une atteinte de l'intégrité cellulaire. La pollution de l'air est associée à la présence de fine particules ayant une taille inférieure à 10 $\mu$M (PM10-like) mais également à la présence de particules de petites tailles inférieures ou égales à 2,5 $\mu$M (PM2.5-

like). Leurs tailles étant plus petites que celles des pores de la peau, les PM2.5-like peuvent directement induire des dommages.

Protocole:

Les kératinocytes humains normaux sont cultivés dans du milieu sans sérum puis traités avec un extrait de *Schinus molle* selon l'exemple 1 à 0,1% dilué dans le milieu de culture pendant 48h à raison de 2 applications par jour. Le contrôle non traité est réalisé dans les mêmes conditions. Au bout de 48h, des particules polluantes PM2.5-like sont appliquées sur les cellules à la concentration de 100 µg/ml pendant 24h avec une seule application.

Le lendemain, le milieu extracellulaire est prélevé afin de mesurer l'activité enzymatique de la lactate déshydrogénase en utilisant le protocole précis et détaillé du kit Lactate Dehydrogenase Activity Assay Kit (Sigma-aldrich, MAK066). Chaque condition est réalisée en triplicat.

Résultats:

Les résultats présentés dans la Figure 2 obtenus *in vitro* montrent que l'extrait de *Schinus molle* selon l'exemple 1 à 0,1% protège, de manière hautement significative, les kératinocytes soumis à un stress aux particules PM2.5-like à la dose de 100 µg/ml (-11% de LDH libérée comparé au traitement PM2.5-like 100 µg/ml).

Exemple 7 : Evaluation de l'effet de l'extrait de Schinus molle selon l'exemple 1 sur le niveau d'expression de marqueurs épigénétiques et d'ARNm impliqués dans la fonction barrière de la peau.

Le but de cette étude est de déterminer l'effet de l'extrait de Schinus molle selon l'exemple 1 à 0,1%, sur les niveaux d'expression de deux longs ARN non codants (modulateurs épigénétiques de la fonction barrière de la peau): TINCR (Tissue differentiation-Inducing Non-protein Coding RNA) et DANCR (Differentiation Antagonizing Non-protein Coding RNA) ainsi que les niveaux d'expression d'ARNm codant pour des protéines impliquées dans la fonction barrière de la peau comme: E-cadherin (CDH1), Involucrin (IVL), Transglutaminase-1 (TGM1) et Caspase 14 (CASP14) dans des kératinocytes humains normaux.

Protocole:

Les kératinocytes humains normaux sont cultivés dans du milieu sans sérum puis traités avec un extrait de *Schinus molle* selon l'exemple 1 à 0,1% dilué dans le milieu de culture pendant 48h à raison de 2 applications par jour. Le contrôle non traité est réalisé dans les mêmes conditions.

Les ARNs totaux sont extraits à l'aide du kit d'extraction miRVana (Ambion, AM1561), puis une transcription inverse est réalisée à l'aide du kit High Capacity cDNA reverse transcription (Applied Biosystem, 4374966) pour chacune des conditions. Une PCR quantitative en temps réel est réalisée en utilisant des TaqMan® Gene Expression Assays spécifiques de DANCR (Applied Biosystem: 4426961 ; Hs03653830_g1), TINCR, TGM1, IVL et CASP14 (Applied Biosystem: 4331182 ; Hs00542141_m1, Hs01070310_m1, HsO0846307_s1, Hs00201637_m1) et le tampon de réaction TaqMan® Gene Expression Master Mix (Applied Biosystem: 4369514), dans le thermocycleur StepOne Plus (Applied Biosystem). Le TaqMan® Gene Expression Assays spécifique du 18S est utilisé comme contrôle endogène (Applied Biosystem: 4331182 ; Hs99999901_s1). La quantification relative de l'expression des différents ARNm est réalisée par la méthode comparative des Ct. Les résultats sont donnés en pourcentage d'augmentation du niveau d'expression par rapport à la condition contrôle.

Résultats (Tableau) et conclusions:

| Fonction Barrière de la Peau | | | | | |
|---|---|---|---|---|---|
| Marqueurs biologiques épigénétiques | | Marqueurs biologiques: ARNm | | | |
| DANCR | TINCR | E-Cadherin | Involucrin | Transglutaminase-1 | Caspase 14 |
| -26% | +65% | +34% | +283% | +185% | +152% |

Les résultats répertoriés dans le Tableau ci-dessus montrent que l'extrait de *Schinus molle* selon l'exemple 1 à 0,1% module positivement la différenciation épidermique, *in vitro.* L'extrait de *Schinus molle* selon l'exemple 1 à 0,1% augmente, *in vitro,* l'expression des ARNm codant pour la E-cadhérine, l'involucrine, la transglutaminase-1 et la Caspase 14, protéines impliquées dans la fonction barrière de la peau.

Exemple 8 : Evaluation de l'effet de l'extrait de Schinus molle selon l'exemple 1 sur la cohésion cellulaire de la barrière cutanée par l'étude de la Claudine 1.

Le but de cette étude est d'évaluer l'effet de l'extrait de *Schinus molle* selon l'exemple 1 à 1% sur l'expression de la protéine Claudine 1 impliquée dans les jonctions serrées de l'épiderme. La Claudine 1 forme un gradient d'expression dans l'épiderme. Les jonctions serrées sont des structures dynamiques qui assurent la cohésion cellulaire et la fonction barrière de la peau.

Protocole:

Des biopsies de peau humaine de 6 mm de diamètre sont maintenues en culture ex *vivo* en présence d'un milieu spécifique (DMEM 1g/L, HAMF12, SVF et antibiotiques) sur des inserts déposés dans des plaques 6 puits. Les biopsies sont cultivées pendant 48h et reçoivent 2 applications par jour d'extrait de *Schinus molle* selon l'exemple 1 dilué à 1% dans du PBS 1X ou de PBS 1X pour la condition contrôle. Les biopsies sont ensuite fixées dans le formaldéhyde puis incluses dans la paraffine. Des coupes de peau de 4 $\mu$m d'épaisseur sont ensuite réalisées. L'immuno-marquage est réalisé à l'aide d'un anticorps polyclonal de lapin spécifique de la Claudine 1 (Abcam, ab15098) puis d'un anticorps secondaire anti-lapin couplé à un fluorochrome (Invitrogen, A21206). Les biopsies sont alors examinées au microscope à Epi-fluorescence (Zeiss Axiovert 200M microscope). Une quantification de la fluorescence, à l'aide du logiciel Volocity® image analysis software (PerkinElmer, Inc.) a été réalisée à partir des photographies obtenues.

Résultats et conclusions:

Le traitement avec l'extrait de *Schinus molle* selon l'exemple 1 à 1% permet d'observer une augmentation hautement significative de l'expression de la Claudine 1 de 128,2%, comparé à la condition contrôle traitée avec du PBS 1X, pour l'étude ex *vivo* (test t de Student, n=3 +/- sem (sem : erreur type de la moyenne)).
Le traitement par l'extrait de *Schinus molle* selon l'exemple 1 à 1% provoquerait une meilleure cohésion des couches les plus différenciées de l'épiderme et renforcerait ainsi la fonction barrière cutanée.

Exemple 9 : Evaluation de l'effet de l'extrait de Schinus molle selon l'exemple 1 sur la cohésion cellulaire sous tendant la fonction barrière cutanée par l'étude de l'acide hyaluronique sur biopsies ex vivo.

Le but de cette étude est d'évaluer l'effet de l'extrait de Schinus molle selon l'exemple 1 à 1% sur l'expression de l'acide hyaluronique impliqué dans l'établissement de la barrière cutanée. L'acide hyaluronique est abondant dans les matrices extracellulaires et contribue également à l'hydrodynamisme de l'épiderme en modulant la différenciation épidermique, et l'amélioration de la réparation de la peau.

Protocole:

Des biopsies de peau humaine de 6 mm de diamètre sont maintenues en culture ex *vivo* en présence d'un milieu spécifique (DMEM 1 g/L, HAMF12, SVF et antibiotiques) sur des inserts déposés dans des plaques 6 puits. Les biopsies sont cultivées pendant 48h et reçoivent 2 applications par jour d'extrait de *Schinus molle* selon l'exemple 1 dilué à 1% dans du PBS 1X ou de PBS 1X pour la condition contrôle. Les biopsies sont ensuite fixées dans le formaldéhyde puis incluses dans la paraffine. Des coupes de peau de 4 $\mu$m d'épaisseur sont ensuite réalisées. La détection de l'acide hyaluronique est réalisée à l'aide d'une protéine de liaison à l'acide hyaluronique biotinylée (Coger - Seikagaki america, 400-763-1A) puis d'une streptavidine couplée à un fluorochrome (Invitrogen, S32354). Les biopsies sont alors examinées au microscope à Epi-fluorescence (Zeiss Axiovert 200M microscope). Une quantification de la fluorescence, à l'aide du logiciel Volocity® image analysis software (PerkinElmer, Inc.) a été réalisée à partir des photographies obtenues.

Résultats et conclusions:

Le traitement avec l'extrait de *Schinus molle* selon l'exemple 1 à 1% permet d'observer une augmentation très significative de l'acide hyaluronique dans le derme et l'épiderme de 58,9%, comparé à la condition contrôle traitée avec du PBS 1X, pour l'étude ex *vivo* (test t de Student, n=3 +/- sem).

Le traitement par l'extrait de *Schinus molle* selon l'exemple 1 à 1% permettrait une amélioration de la fonction barrière cutanée.

Exemple 10 : Evaluation de l'effet protecteur de l'extrait de Schinus molle selon l'exemple 1 sur la teneur en lipides des kératinocytes en culture.

Le but de cette étude est de déterminer l'effet de l'extrait de Schinus molle selon l'exemple 1 à 0,1%, sur la teneur en lipides des kératinocytes, qui sous tend la fonction barrière cutanée.

Protocole:

Les kératinocytes humains normaux sont cultivés dans du milieu sans sérum puis traités avec un extrait de *Schinus molle* selon l'exemple 1 à 0,1% dilué dans le milieu de culture pendant 48h à raison de 2 applications par jour. Le contrôle non traité est réalisé dans les mêmes conditions. Au bout de 48h, les cellules sont fixées avec du formaldéhyde à 3,7% pendant 10 minutes (Sigma-aldrich, F1635). Après rinçage au PBS, les cellules sont mises au contact de Nile Red à 100 nM pendant 10 minutes (Sigma-aldrich, N3013) avant d'être rincées au PBS 1X. Les cellules sont alors examinées au microscope à Epi-fluorescence (Zeiss Axiovert 200M microscope).
Une quantification de la fluorescence, à l'aide du logiciel Volocity® image analysis software (PerkinElmer, Inc.) a été réalisée à partir des photographies obtenues.

Résultats :

Les observations microscopiques montrent une fluorescence hautement significative avec l'extrait de *Schinus molle* selon l'exemple 1 à 0,1%, sur la synthèse des lipides des kératinocytes (+102% comparé au non traité (selon le test t de Student, n=3 +/- sem)).
Le traitement par l'extrait de *Schinus molle* selon l'exemple 1 à 0,1% permettrait un renforcement de la fonction barrière cutanée.

Exemple 11 : Evaluation de l'effet de l'extrait de Schinus molle selon l'exemple 1 sur la cohésion cellulaire de la barrière cutanée par l'étude des lipides de l'épiderme sur biopsies ex vivo.

Le but de cette étude est d'évaluer l'effet de l'extrait de Schinus molle selon l'exemple 1 à 1% sur le contenu de l'épiderme en lipides. Les lipides composant l'épiderme ont un rôle important dans le maintien de la fonction barrière et dans sa protection face aux agressions extérieures.

Protocole:

Des biopsies de peau humaine de 6 mm de diamètre sont maintenues en culture ex *vivo* en présence d'un milieu spécifique (DMEM 1 g/L, HAMF12, SVF et antibiotiques) sur des inserts déposés dans des plaques 6 puits. Les biopsies sont cultivées pendant 48h et reçoivent 2 applications par jour d'extrait de *Schinus molle* selon l'exemple 1 dilué à 1% dans du PBS 1X ou de PBS 1X pour la condition contrôle. Les biopsies sont ensuite fixées dans le formaldéhyde puis incluses dans la paraffine. Des coupes de peau de 4 $\mu$m d'épaisseur sont ensuite réalisées. La détection du contenu lipidique de l'épiderme est réalisée à l'aide d'une solution de Nile Red à 100 nM (Sigma-aldrich, N3013) puis d'une incubation dans un tampon alcalin. Les biopsies sont alors examinées au microscope à Epi-fluorescence (Zeiss Axiovert 200M microscope).
Une quantification de la fluorescence, à l'aide du logiciel Volocity® image analysis software (PerkinElmer, Inc.) a été réalisée à partir des photographies obtenues.

Résultats et conclusions:

Le traitement avec l'extrait de *Schinus molle* selon l'exemple 1 à 1% permet d'observer une augmentation très significative des lipides dans l'épiderme de 41%, comparé à la condition contrôle traitée avec du PBS 1X, pour l'étude ex *vivo* (test t de Student, n=3 +/- sem).
Le traitement par l'extrait de *Schinus molle* selon l'exemple 1 à 1% permettrait un renforcement de la fonction barrière cutanée.

Exemple 12 : Evaluation de l'effet de l'extrait de Schinus molle selon l'exemple 1 sur la fonction barrière de la peau après un stress SDS.

Le but de cette étude est d'évaluer, in vitro, sur tissus reconstruits, l'effet de l'extrait de Schinus molle selon l'exemple 1 à 1%, sur la perméabilité de la peau après un stress SDS en utilisant un colorant fluorescent.

Protocole:

Des épidermes humains reconstruits (RHE) sont traités, à partir du jour 10 post-reconstruction, en application topique avec un extrait de *Schinus molle* selon l'exemple 1 dilué à 1% dans du PBS 1X pendant 48h à raison de 2 applications par jour. Le contrôle est traité avec du PBS 1X dans les mêmes conditions.

Les RHE, sont ensuite traités, le 12ème jour post-reconstruction, avec du SDS à 0,15% pendant 3h puis rincés 5 fois avec du PBS 1X. Les RHE sont alors traités avec 1 mM de lucifer yellow (Fluka, 62644), colorant fluorescent, pendant 2h. Les RHE sont rincés au PBS 1X et ensuite fixés dans du formaldéhyde puis inclus dans la paraffine. Des coupes de peau de 4 $\mu$m d'épaisseur sont réalisées et déposées sur lame. Les lames sont alors déparaffinées et passées dans plusieurs bains d'alcool successifs.

La pénétration du colorant à travers la barrière et dans le tissu est observée au microscope à Epi-fluorescence (Zeiss Axiovert 200M microscope). La diffusion de la fluorescence au travers de l'épiderme est évaluée à partir des photographies obtenues à l'aide du logiciel Volocity® image analysis software (PerkinElmer, Inc.).

Résultats et conclusions:

Les RHE prétraités avec un extrait de *Schinus molle* selon l'exemple 1 à 1% et stressés avec le SDS à 0,15%, laissent moins pénétrer le colorant. On observe une diminution significative de 16% de la diffusion du colorant dans l'épiderme comparé à la condition traitée PBS 1X et stressée avec du SDS 0,15% (test t de Student, « SDS 0,15% » n=4, « 1%+SDS » n=7, moyenne $\pm$ sem).

Le traitement par l'extrait de *Schinus molle* selon l'exemple 1 à 1% permettrait un renforcement de la fonction barrière cutanée.

Exemple 13 : Effet d'un extrait de Schinus molle formulé selon l'exemple 3 sur la régulation de la sécrétion de sébum - Etude in vivo

Objectif : étude in vivo de l'effet d'un extrait de Schinus molle formulé selon l'exemple 3 sur la régulation de la sécrétion de sébum chez des volontaires sains contre la composition placebo.
Nombre de volontaires sains : 10 (âgés de 24 à 46 ans).
Etude : étude en double aveugle contre placebo.
Produits testés : Extrait de *Schinus molle,* formulé à 1% dans une composition (Formule référence #TCAAE08) selon l'exemple 3 *versus* la composition placebo.
Nombre d'application : 2 applications par jour, matin et soir, à une dose de 2 mg/cm$^2$.
Durée du test : 3 semaines.
Visites de contrôle : J0, J0-t1h, J7 and J21.

Mesures :

La sécrétion de sébum physiologique, c'est-à-dire dans des conditions non pathologiques, est évaluée par l'application de Sebutape®, un adhésif blanc opaque perméable au sébum, sur la peau des volontaires. Les zones qui sont au contact du sébum forment des « spots » transparents. L'analyse quantitative de la surface et du nombre de spots présents sur les Sebutapes est réalisée à l'aide du logiciel Quantiseb®. La taille des pores est évaluée indépendamment sur photos à l'aide d'un DermaTop®.

Analyses statistiques : test " t " de Student.

Résultats :

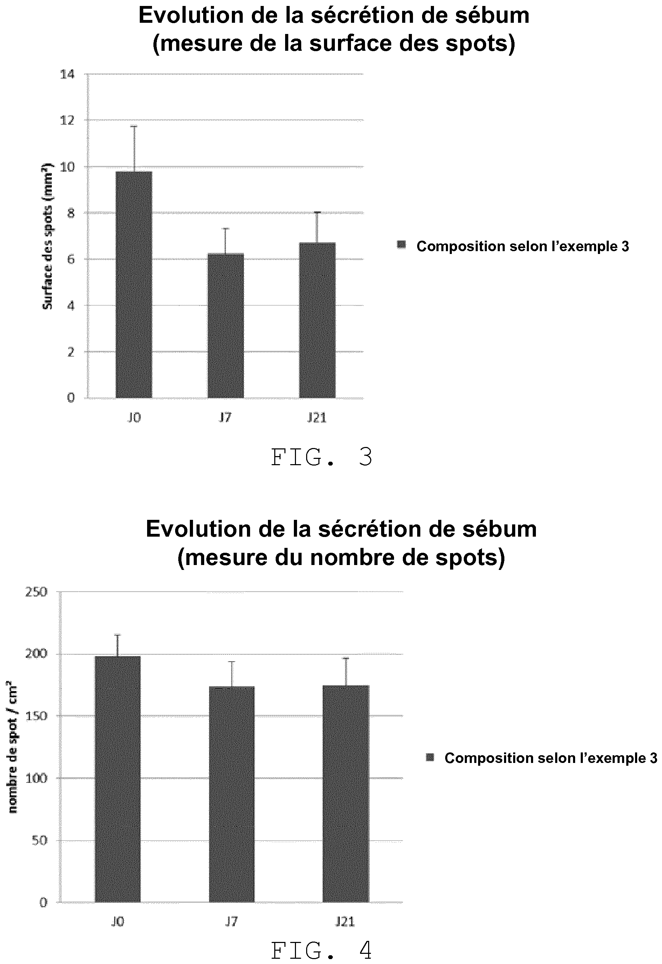

Après application de la composition (référence #TCAAE08) comprenant un extrait de *Schinus molle* formulé à 1% selon l'exemple 3, l'analyse montre une diminution statistiquement significative à J7 et J21 de la surface des spots (Figure 3 et Tableau 3) et du nombre de spots/cm$^2$ (Figure 4 et Tableau 4).

Tableau 3 : Evolution de la sécrétion de sébum (mesure de la surface des spots) après application de la composition contenant l'extrait de *Schinus molle* à 1% selon l'exemple 3

| Traitement | Temps | Moyenne (mm$^2$) | Sem | p | % de changement | % de volontaires ayant eu une diminution de la sécrétion de sébum |
|---|---|---|---|---|---|---|
| Placebo | J7-J0 | 3,26 | 0, 97 | | | |
| Formule référence #TCAAE08 (Extrait de *Schinus molle* à 1% selon exemple 3) | J7-J0 | -3,59 | 1,1 | 0,0004*** | -81% | 100 |
| Placebo | J21-J0 | 3, 04 | 1,19 | | | |
| Formule référence #TCAAE08 (Extrait de *Schinus molle* à 1% selon exemple 3) | J21-J0 | -3,09 | 1, 46 | 0,0051** | -72,6% | 90 |
| **: très significatif; ***: hautement significatif avec le test de Student; moyenne n=10 +/- sem (erreur type de la moyenne). | | | | | | |

Tableau 4 : Evolution de la sécrétion de sébum (mesure du nombre de spots) après application de la composition contenantl'extrait de Schinus molle à 1% selon l'exemple 3

| Traitement | Temps | Moyenne (nombre/cm$^2$) | sem | p | % de changement | % de volontair es ayant eu une diminution du nombre des spots |
|---|---|---|---|---|---|---|
| Placebo | J7-J0 | 50,32 | 15,09 | | | |
| Formule référence #TCAAE08 (Extrait de *Schinus molle* à 1% selon exemple 3) | J7-J0 | -24,06 | 14,58 | 0,0061** | -40% | 90 |
| Placebo | J21-J0 | 45, 68 | 11,23 | | | |
| Formule référence #TCAAE08 (Extrait de *Schinus molle* à 1% selon exemple 3) | J21-J0 | -22, 96 | 16,86 | 0,0033*** | -37,2% | 80 |
| **: très significatif; ***: hautement significatif avec le test de Student; moyenne n=10 +/- sem (erreur type de la moyenne). | | | | | | |

Par ailleurs, les photos du DermaTop (non représentées) montrent un effet visible sur la réduction de la taille des pores et de leur nombre dès 1 heure après application (J0-t1h) de la composition (formule référence #TCAAE08) comprenant un extrait de *Schinus molle* selon l'exemple 3 comparé au placebo.

Exemple 14 : Effet d'un extrait de Schinus molle formulé selon l'exemple 3 sur l'apparence des rides - Etude in vivo

Objectif : étude de l'effet d'un extrait de *Schinus molle* formulé selon l'exemple 3 sur l'apparence des rides contre la composition placebo.
Nombre de volontaires : 14 dont 4 sorties d'essais. Le test a été analysé sur 10 volontaires âgés de 52 à 64 ans.
Etude : étude en double aveugle contre placebo.

Produits testés : Extrait de *Schinus molle,* formulé à 1% dans une composition (Formule référence #TCAAE08) selon l'exemple 3 *versus* la composition placebo.

Nombre d'application : 2 applications par jour, matin et soir, à une dose de 2 mg/cm$^2$.

Durée du test : 8 semaines.

Visites de contrôle : J0 et J56.

Mesures :

Le nombre et la profondeur des rides sont analysés sur des répliques de silicone faites au niveau de la patte d'oie à l'aide du logiciel Quantirides®. L'apparence visuelle est évaluée par photos couleur de la patte d'oie à l'aide du banc photo HeadScan®.

Analyses statistiques : en fonction de la normalité de distribution des données, le test " t " de Student ou le test de Wilcoxon ont été utilisés.

Résultats :

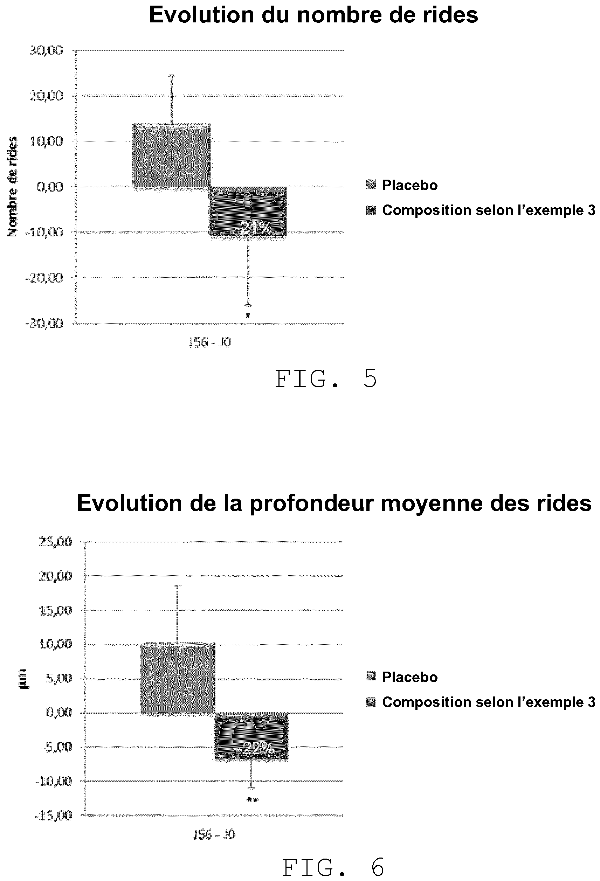

Après application de la composition (référence #TCAAE08) comprenant un extrait de *Schinus molle* formulé à 1% selon l'exemple 3, on observe une diminution statistiquement significative à J56 du nombre de rides (Figure 5 et Tableau 5) et de la profondeur moyenne des rides (Figure 6 et Tableau 5), comparé au placebo.

Tableau 5 : Etude de l'effet d'un extrait de *Schinus molle* formulé selon l'exemple 3 sur le nombre de rides et leur profondeur moyenne

| | Traitement | Temps | Moyenne | sem | p | % de changement | % de volontaires améliorés |
|---|---|---|---|---|---|---|---|
| Nombre de rides | Placebo | J56-J0 | 13,9 | 10, 39 | 0,0304* | -21,16 | 70% (7/10) |
| | Formule référence #TCAAE08 comprenant un extrait de *Schinus molle* selon exemple 3, formulé à 1% | J56-J0 | -10,8 | 15,35 | | | |
| Profondeur moyenne (μm) | Placebo | J56-J0 | 10,28 | 8, 33 | 0,0068** | -22,29 | 80% (8/10) |
| | Formule référence #TCAAE08 comprenant un extrait de *Schinus molle* selon exemple 3, formulé à 1% | J56-J0 | -6,59 | 4, 4 | | | |
| * : significatif; ** : très significatif; avec le test de Student ou le test de Wilcoxon (test sélectionné en fonction de la normalité de distribution des données); moyenne n=10 +/- sem (erreur type de la moyenne). | | | | | | | |

De plus, les photos couleur (non représentées) montrent une réduction apparente des rides à J56 après application de la composition (référence #TCAAE08) comprenant un extrait de *Schinus molle* selon l'exemple 3, formulé à 1% comparé au placebo.

Exemple 15 : Protocole test d'un extrait de Schinus molle selon l'exemple 3, formulé à 1%: Effet hydratant - Etude in vivo

Objectif : étude de l'effet hydratant d'un extrait de *Schinus molle* selon l'exemple 3, formulé à 1% contre la composition placebo.

Nombre de volontaires : 10 (âgés de 24 à 40 ans).

Etude : étude en double aveugle contre placebo.

Produits testés : Extrait de *Schinus molle,* formulé à 1% dans une composition (Formule référence #TCAAE08) selon l'exemple 3 *versus* la composition placebo.

Nombre d'application : 1 application à une dose de 2 mg/cm$^2$. Durée du test : 3h.

Visites de contrôle : T0, T30min, T1h, T1h30, T2h, T2h30, T3h. Mesures : mesure quantitative de l'hydratation à l'aide d'un cornéomètre corneometer® CM825 et évaluation visuelle par un expert et par les volontaires.

Analyses statistiques : en fonction de la normalité de distribution des données, le test " t " de Student ou le test de Wilcoxon ont été utilisés.

Résultats :

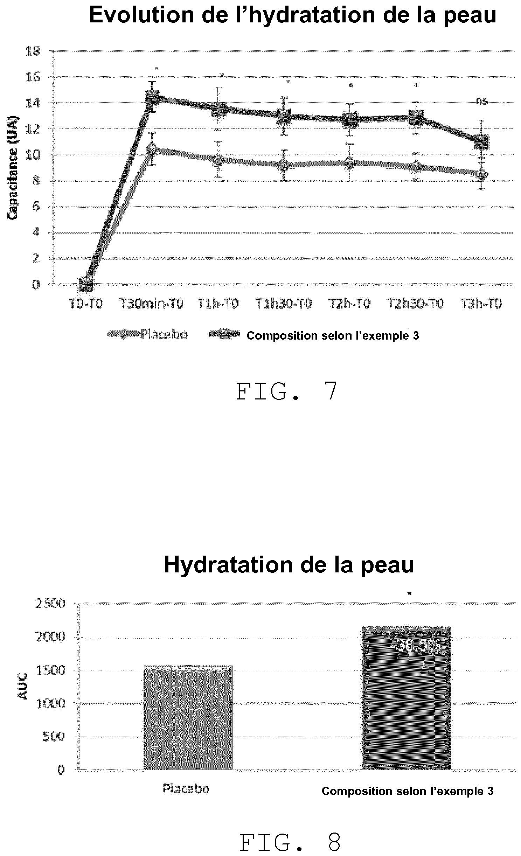

On observe une augmentation significative de l'hydratation (Figure 7 et Tableau 6) dès 30 min après l'application de la composition (référence #TCAAE08) comprenant un extrait de *Schinus molle* selon l'exemple 3, formulé à 1%, comparé au placebo.

Tableau 6 : Etude de l'évolution de l'hydratation de la peau après application d'un extrait de *Schinus molle* formulé selon l'exemple 3

| Traitement | Temps | Moyenne (UA) | sem | p | % de volontaires améliorés |
|---|---|---|---|---|---|
| Placebo | T30min-T0 | 10,43 | 1,26 | 0,0107* | 80% (8/10) |
| Formule référence #TCAAE08 (exemple 3) | | 14,43 | 1, 17 | | |
| Placebo | T1h-T0 | 9,6 | 1,38 | 0,0190* | 70% (7/10) |
| Formule référence #TCAAE08 (exemple 3) | | 13,53 | 1,69 | | |
| Placebo | T1h30-T0 | 9,2 | 1,16 | 0,0263* | 80% (8/10) |
| Formule référence #TCAAE08 (exemple 3) | | 12,97 | 1,44 | | |
| Placebo | T2h-T0 | 9,4 | 1,44 | 0,0352* | 70% (7/10) |
| Formule référence #TCAAE08 (exemple 3) | | 12,7 | 1,23 | | |
| Placebo | T2h30-T0 | 9,1 | 1,02 | 0,0103* | 80% (8/10) |
| Formule référence #TCAAE08 (exemple 3) | | 12,87 | 1,22 | | |
| Placebo | T3h-T0 | 8,53 | 1,22 | 0,0534$^{ns}$ | 70% (7/10) |
| Formule référence #TCAAE08 (exemple 3) | | 11,03 | 1,64 | | |
| ns : non significatif; * : significatif avec le test de Student ou le test de Wilcoxon (test sélectionné en fonction de la normalité de distribution des données); moyenne n=10 +/- sem (erreur type de la moyenne). | | | | | |

De plus, le calcul des aires sous courbes (AUC) montre un effet hydratant significatif sur la durée de l'étude (Figure 8 et Tableau 7).

Tableau 7 : Etude de l'effet hydratant d'un extrait de *Schinus molle* formulé selon l'exemple 3

| Traitement | AUC | sem | p | % de changement | % de volontaires améliorés |
|---|---|---|---|---|---|
| Placebo | 1560 | 189,24 | 0,0129* | 38,49% | 70% (7/10) |
| Formule référence #TCAAE08 | 2160,5 | 211,43 | | | |
| * : significatif avec le test de Student; AUC n=10 +/- sem (erreur type de la moyenne). | | | | | |

Bien entendu, l'invention ne se limite pas aux modes de réalisation et aux exemples présentés ci-dessus et l'homme du métier, grâce à des opérations de routine, pourra être amené à réaliser d'autres modes de réalisation non décrits explicitement, qui entrent dans le cadre large de l'invention.

**Revendications**

1.  Extrait hydro-alcoolique des parties aériennes de *Schinus molle* **caractérisé en ce qu'**il est obtenu à partir des feuilles de *Schinus molle* non débarrassées des petites tiges portant les feuilles, séchées et broyées, par extraction solide-liquide, sous agitation, de 5% à 10% en poids de matière solide par rapport au poids total mis en œuvre dans un solvant hydro-alcoolique, l'alcool étant choisi parmi l'éthanol ou le propanediol, dans une proportion comprise entre 60% et 80% en poids d'alcool par rapport au poids total du solvant, à une température comprise entre 20°C et 70°C pendant une durée comprise entre 1 heure et 2 heures, et par séparation des phases liquide et solide, de manière à éliminer la phase solide et récupérer un extrait de *Schinus molle* liquide, ledit extrait comprenant majoritairement de la quercitrine et de la miquelianine parmi les polyphénols extraits, en une concentration respectivement d'au moins 0,04% de quercitrine en poids du poids total de l'extrait et au moins 0,02% de miquelianine en poids du poids total de l'extrait, les autres polyphénols extraits pris indépendamment les uns des autres étant présents à une concentration inférieure à 0,01% (100 ppm) en poids du poids total de l'extrait.

2.  Extrait selon la revendication 1, **caractérisé en ce que**, lorsque l'alcool est l'éthanol, l'extrait de Schinus molle liquide obtenu est séché de manière à obtenir un extrait de Schinus molle solide.

3.  Procédé d'obtention d'un extrait de Schinus molle selon l'une des revendications 1 ou 2, **caractérisé en ce qu'**il comprend les étapes suivantes selon lesquelles :

    a) on recueille les feuilles non débarrassées des petites tiges portant les feuilles de *Schinus molle* ;
    b) on disperse les feuilles non débarrassées des petites tiges portant les feuilles de *Schinus molle* recueillies, séchées et broyées, dans une proportion de 5% à 10% en poids de matière solide par rapport au poids total mis en œuvre, dans un solvant hydro-alcoolique, l'alcool étant choisi parmi l'éthanol ou le propanediol, dans une proportion comprise entre 60% et 80% en poids d'alcool par rapport au poids total du solvant ;
    c) on réalise une extraction solide-liquide, sous agitation, à une température comprise entre 20°C et 70°C pendant une durée comprise entre 1 heure et 2 heures ;
    d) on sépare les phases liquide et solide de manière à éliminer la phase solide et récupérer un extrait hydro-alcoolique de *Schinus molle* liquide comprenant majoritairement de la quercitrine et de la miquelianine parmi les polyphénols extraits, en une concentration respectivement d'au moins 0,04% de quercitrine en poids du poids total de l'extrait et au moins 0,02% de miquelianine en poids du poids total de l'extrait, les autres polyphénols extraits pris indépendamment les uns des autres étant présents à une concentration inférieure à 0,01% (100 ppm) en poids du poids total de l'extrait; et
    e) éventuellement, lorsque l'alcool est l'éthanol, on sèche l'extrait de Schinus molle liquide obtenu de manière à obtenir un extrait de Schinus molle solide.

4.  Procédé selon la revendication 3, **caractérisé en ce que** l'extrait de Schinus molle liquide obtenu est purifié par microfiltration, ultrafiltration et/ou nanofiltration pour concentrer l'extrait en quercitrine et miquelianine par rapport aux protéines et polysaccharides également extraits.

5.  Composition cosmétique, **caractérisée en ce qu'**elle comprend, à titre d'agent actif protecteur, une quantité efficace d'un extrait de Schinus molle selon l'une des revendications 1 ou 2, et un excipient physiologiquement acceptable.

**6.** Composition selon la revendication 5, **caractérisée en ce que** l'extrait de Schinus molle est présent dans la composition à une concentration de 0,001 à 1% en poids par rapport au poids total de la composition, préférentiellement de 0,01 à 1%.

**7.** Composition selon l'une des revendications 5 ou 6, **caractérisée en ce qu'**elle est formulée pour être appliquée topiquement sur la peau.

**8.** Composition selon l'une des revendications 5 à 7, pour son utilisation pour améliorer la fonction barrière de la peau et protéger la peau contre les polluants atmosphériques.

**9.** Utilisation d'une composition selon l'une des revendications 5 7, pour hydrater la peau et lutter contre l'apparition des signes du vieillissement cutané.

**10.** Utilisation d'une composition selon l'une des revendications 5 à 7, pour réduire la taille des pores de la peau.

**11.** Utilisation d'une composition selon l'une des revendications 5 à 7, pour limiter la sécrétion de sébum.

**Patentansprüche**

**1.** Hydroalkoholischer Extrakt aus den oberirdischen Teilen von *Schinus molle,* **dadurch gekennzeichnet, dass** er aus den Blättern von *Schinus molle* gewonnen wird, die nicht von den die Blätter tragenden kleinen Stängeln befreit sind, getrocknet und gemahlen, durch Fest-Flüssig-Extraktion unter Rühren, von 5 bis 10 Gew.-% Feststoff, bezogen auf das Gesamtgewicht, das in einem hydroalkoholischen Lösungsmittel verwendet wird, wobei der Alkohol aus Ethanol oder Propandiol gewählt wird, in einem Anteil von 60 bis 80 Gew.-% Alkohol, bezogen auf das Gesamtgewicht des Lösungsmittels, bei einer Temperatur zwischen 20°C und 70°C für einen Zeitraum zwischen 1 Stunde und 2 Stunden, und durch Trennen der flüssigen und festen Phase, um die feste Phase zu entfernen und einen Extrakt aus flüssigem *Schinus molle* zu gewinnen, wobei der Extrakt hauptsächlich Quercitrin und Miquelianin unter den extrahierten Polyphenolen in einer Konzentration von jeweils mindestens 0,04 Gew.-% Quercitrin enthält, bezogen auf das Gesamtgewicht des Extrakts und mindestens 0,02 Gew.-% Miquelianin, bezogen auf das Gesamtgewicht des Extrakts, wobei die anderen extrahierten Polyphenole, die unabhängig voneinander entnommen werden, in einer Konzentration von weniger als 0,01 Gew.-% (100 ppm) des Gesamtgewichts des Extrakts vorhanden sind.

**2.** Extrakt nach Anspruch 1, **dadurch gekennzeichnet, dass**, wenn der Alkohol Ethanol ist, der resultierende flüssige Extrakt von *Schinus molle* getrocknet wird, um einen festen Extrakt von *Schinus molle* zu erhalten.

**3.** Verfahren zur Gewinnung eines Extraktes aus *Schinus molle* nach einem der Ansprüche 1 oder 2, **dadurch gekennzeichnet, dass** es die folgenden Schritte umfasst:

a) die nicht von den Stängeln befreiten Blätter des *Schinus molle* werden gesammelt;
b) die gesammelten, getrocknete, und gemahlenen nicht von den Stängeln befreiten Blätter des *Schinus molle* werden in einem Anteil von 5 bis 10 Gew.-% Feststoff, bezogen auf das verwendete Gesamtgewicht in einem hydroalkoholischen Lösungsmittel dispergiert, wobei der Alkohol aus Ethanol oder Propandiol gewählt wird, in einem Anteil von zwischen 60 und 80 Gew.-% Alkohol, bezogen auf das Gesamtgewicht des Lösungsmittels;
c) eine Fest-Flüssig-Extraktion wird unter Rühren bei einer Temperatur zwischen 20°C und 70°C für einen Zeitraum zwischen 1 Stunde und 2 Stunden durchgeführt;
d) sie flüssige und die feste Phase werden getrennt, um die feste Phase zu entfernen und einen hydroalkoholischen Extrakt aus flüssigem *Schinus molle* zu gewinnen, der hauptsächlich Quercitrin und Miquelianin unter den extrahierten Polyphenolen enthält, in einer Konzentration von mindestens 0,04 Gew.-% Quercitrin, bezogen auf das Gesamtgewicht des Extrakts und mindestens 0,02 Gew.-% Miquelianin, bezogen auf das Gesamtgewicht des Extrakts, wobei die anderen extrahierten Polyphenole, die unabhängig voneinander eingenommen werden, in einer Konzentration von weniger als 0,01 Gew.-% (100 ppm) des Gesamtgewichts des Extrakts vorhanden sind; und
e) gegebenenfalls, wenn der Alkohol Ethanol ist, wird der erhaltene flüssige Extrakt von *Schinus molle* getrocknet, um einen festen Extrakt von *Schinus molle* zu erhalten.

**4.** Verfahren nach Anspruch 3, **dadurch gekennzeichnet, dass** der erhaltene flüssige Extrakt von *Schinus molle* durch Mikrofiltration, Ultrafiltration und/oder Nanofiltration gereinigt wird, um den Extrakt hinsichtlich Quercitrin und Miquelianin in Bezug auf die ebenfalls extrahierten Proteine und Polysaccharide zu konzentrieren.

5.  Kosmetische Zusammensetzung, **dadurch gekennzeichnet, dass** sie als schützender Wirkstoff eine wirksame Menge eines Extrakts von *Schinus molle* nach einem der Ansprüche 1 oder 2 und einen physiologisch verträglichen Trägerstoff enthält.

6.  Zusammensetzung nach Anspruch 5, **dadurch gekennzeichnet, dass** der Extrakt von *Schinus molle* in der Zusammensetzung in einer Konzentration von 0,001 bis 1 Gew.-%, bezogen auf das Gesamtgewicht der Zusammensetzung, vorzugsweise 0,01 bis 1% enthalten ist.

7.  Zusammensetzung nach einem der Ansprüche 5 oder 6, **dadurch gekennzeichnet, dass** sie zur örtlichen Anwendung auf der Haut konzipiert ist.

8.  Zusammensetzung nach einem der Ansprüche 5 bis 7 zur Verwendung bei der Verbesserung der Barrierefunktion der Haut und zum Schutz der Haut vor Luftschadstoffen.

9.  Verwendung einer Zusammensetzung nach einem der Ansprüche 5 bis 7, um die Haut mit Feuchtigkeit zu versorgen und das Auftreten von Zeichen der Hautalterung zu bekämpfen.

10. Verwendung einer Zusammensetzung nach einem der Ansprüche 5 bis 7, um die Porengröße der Haut zu reduzieren.

11. Verwendung einer Zusammensetzung nach einem der Ansprüche 5 bis 7, um die Talgsekretion zu begrenzen.

**Claims**

1.  Hydroalcoholic extract of the aerial parts of *Schinus molle,* **characterised in that** it is obtained from *Schinus molle* leaves not rid of the small stems carrying the leaves, dried and crushed, by solid-liquid extraction, with stirring, of 5% to 10% by weight of solid matter relative to the total weight used in a hydroalcoholic solvent, the alcohol being selected from ethanol or propanediol, in a proportion of between 60% and 80% by weight of alcohol relative to the total weight of the solvent, at a temperature of between 20°C and 70°C for a period of between 1 hour and 2 hours, and by separation of the liquid and solid phases, so as to remove the solid phase and recover a liquid *Schinus molle* extract, said extract comprising mainly quercitrin and miquelianin among the extracted polyphenols, in a concentration of at least 0.04% of quercitrin by weight of the total weight of the extract and at least 0.02% of miquelianin by weight of the total weight of the extract, the other extracted polyphenols taken independently of each other being present at a concentration of less than 0.01% (100 ppm) by weight of the total weight of the extract.

2.  Extract according to claim 1, **characterised in that** when the alcohol is ethanol, the liquid *Schinus molle* extract obtained is dried so as to obtain a solid *Schinus molle* extract.

3.  Process for obtaining a *Schinus molle* extract according to any one of claims 1 or 2, **characterised in that** it comprises the following steps, wherein:

    a) the leaves not rid of the small stems carrying the *Schinus molle* leaves are collected;
    b) the collected leaves not rid of the small stems carrying the *Schinus molle* leaves, dried and crushed, are dispersed in a proportion of 5% to 10% by weight of solid matter relative to the total weight used in a hydroalcoholic solvent, the alcohol being selected from ethanol or propanediol, in a proportion of between 60% and 80% by weight of alcohol relative to the total weight of the solvent;
    c) a solid-liquid extraction is performed, with stirring, at a temperature of between 20°C and 70°C for a period of between 1 hour and 2 hours;
    d) the solid and liquid phases are separated so as to eliminate the solid phase and recover a hydroalcoholic liquid *Schinus molle* extract comprising mainly quercitrin and miquelianin among the extracted polyphenols, in a concentration, respectively, of at least 0.04% of quercitrin by weight of the total weight of the extract and at least 0.02% of miquelianin by weight of the total weight of the extract, the other extracted polyphenols taken independently of each other being present at a concentration of less than 0.01% (100 ppm) by weight of the total weight of the extract; and
    e) optionally, when the alcohol is ethanol, the liquid *Schinus molle* extract obtained is dried so as to obtain a solid *Schinus molle* extract.

4. Process according to claim 3, **characterised in that** the liquid *Schinus molle* extract obtained is purified by microfiltration, ultrafiltration and/or nanofiltration to concentrate the extract in quercitrin and miquelianin relative to proteins and polysaccharides also extracted.

5. Cosmetic composition, **characterised in that** it comprises, as protective active ingredient, an effective amount of a *Schinus molle* extract according to any one of claims 1 or 2, and a physiologically acceptable excipient.

6. Composition according to claim 5, **characterised in that** the *Schinus molle* extract is present in the composition at a concentration of 0.001 to 1% by weight relative to the total weight of the composition, preferably from 0.01 to 1%.

7. Composition according to any one of claims 5 or 6, **characterised in that** it is formulated to be applied topically to the skin.

8. Composition according to any one of claims 5 to 7, for use to improve the barrier function of the skin and to protect the skin against atmospheric pollutants.

9. Use of a composition according to any one of claims 5 to 7, to moisturise the skin and to fight against the appearance of signs of skin aging.

10. Use of a composition according to any one of claims 5 to 7, to reduce the size of the pores of the skin.

11. Use of a composition according to any one of claims 5 to 7, to limit the secretion of sebum.

N=3 moyenne +/- sem; ns: non significatif;*: significatif; **: très significatif; ***: hautement significatif selon le test *t* de Student.

FIG. 1

N=3 moyenne +/- sem; ns: non significatif;*: significatif; **: très significatif; ***: hautement significatif selon le test *t* de Student.

FIG. 2

# Evolution de la sécrétion de sébum
## (mesure de la surface des spots)

■ Composition selon l'exemple 3

FIG. 3

# Evolution de la sécrétion de sébum
## (mesure du nombre de spots)

■ Composition selon l'exemple 3

FIG. 4

# Evolution du nombre de rides

FIG. 5

# Evolution de la profondeur moyenne des rides

FIG. 6

# Evolution de l'hydratation de la peau

FIG. 7

# Hydratation de la peau

FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 2013302419 A
- AR 079996
- JP 04176912 B
- JP 2000247862 B
- FR 2827170
- FR 2837098
- FR 2841781
- FR 2846883
- FR 2817748
- FR 2956818
- FR 2951946
- FR 2944526

### Littérature non-brevet citée dans la description

- **FERREROA et al.** *Acute and subacute toxicity evaluation of ethanolic extract from fruits of Schinus molle in rats,* 2007
- **MACHADO et al.** *Antidepressant-like effect of rutin isolated from the ethanolic extract from Schinus molle L. in mice: Evidence for the involvement of the serotonergic and noradrenergic systems,* 2008
- **AMBASATA et al.** *The useful plants of India,* 1986
- **BELLO et al.** *In vitro pharmacological evaluation of the dichloromethanol extract from Schinus molle L.,* 1998
- **HUERTA et al.** *Toxicity and repellence of aqueous and ethanolic extracts from Schinus molle on elm leaf beetle Xanthogaleruca luteola,* 2010
- **MARTINS MDO et al.** *antimicrobial and toxicological properties of Schinus molle L. essential oils,* 2013
- **BRAS et al.** *Evaluation of the acute dermal exposure of the ethanolic and hexanic extracts from leaves of Schinus molle var. areira in rats,* 2011
- International Cosmetic Ingredient Dictionary and Handbook. 2014
- **POZZO-BALBI et al.** *The triterpenoid acids of Schinus molle,* 1978
- **HÄNSEL et al.** Hagers Handbuch der pharmazeutischen praxis. 1994
- **TERHUNE et al.** *β-spathulene : a new sesquiterpene in Schinus molle oil,* 1974
- **DOMINGUEZ et al.** *Lignoceric acid and other compounds of Schinus molle,* 1971
- **YUEQIN et al.** *Isolation of two triterpenoids and a biflavanone with an anti-inflammatory activity from Schinus molle fruits,* 2003
- **HIERMANN et al.** *Die Untersuchung potentieller Wirkstoffe in epilobium Arten,* 1983
- **MARZOUK et al.** *Antioxidant flavonol glycosides from Schinus molle,* 2006
- **PAN T-L et al.** The impact of urban particulate pollution on skin barrier function and the subsequent drug absorption. *J Dermatol Sci,* 2015
- **LEFEBVRE M.A. et al.** Evaluation of the impact of urban pollution on the quality of skin: a multicentre study in Mexico. *Int J Cosmet Sci.,* 06 Février 2015